# EUROPÄISCHE PATENTANMELDUNG

(11) **EP 3 284 735 A1**
(43) Veröffentlichungstag der Anmeldung: **21.02.2018**
(21) Anmeldenummer: 17188079.2
(22) Anmeldetag: 18.02.2015
(51) Int. Cl.: C07C 67/03, C07C 67/08, C07C 69/82, C08K 5/12

(54) **PENTYL-NONYL-TEREPHTHALATE**

(62) Teilanmeldung aus: 15155562.0
(71) Anmelder: Evonik Degussa GmbH, 45128 Essen (DE)
(72) Erfinder: Woldt, Benjamin, 44892 Bochum (DE); Boeck, Florian, 48143 Münster (DE); Grass, Michael, 45721 Haltern am See (DE); Häger, Harald, 59348 Lüdinghausen (DE)

(57) **Zusammenfassung**

Gemische umfassend Dipentylterephthalat, Dinonylterephthalat und Pentylnonylterephthalat können mit vorgestimmter Verteilung der einzelnen Ester im Gemisch hergestellt und als Weichmacher eingesetzt werden.

## Beschreibung

Die vorliegende Erfindung betrifft Gemische von Terephthalsäureestern, Herstellungsverfahren und Verwendungen solcher Gemische als Weichmacher sowie Mittel, welche entsprechende Gemische enthalten.

Im Bereich der Weichmacher für Polymere werden Terephthalsäureester als Ersatz oder Ergänzung zu Phthalsäureestern bereits seit einigen Jahren eingesetzt. Der kommerziell wichtigste Terephthalsäureester ist dabei seit Jahren Diethylhexylterephthalat, welches vereinfacht oft auch als Dioctylterephthalat bezeichnet wird. Terephthalsäureester, welche Alkoholreste mit weniger oder mehr als 8 Kohlenstoffatomen enthalten, werden im Stand der Technik ebenfalls beschrieben.

Die Terephthalsäureester haben - unter anderem abhängig von der Kohlenstoffanzahl in den Alkoholresten der Esterfunktionen - unterschiedliche Eigenschaften und eignen sich dementsprechend mehr oder weniger stark für unterschiedliche Weichmacheranwendungen. So gelieren kürzerkettige Terephthalsäureester tendenziell bei niedrigeren Temperaturen als ihre längerkettigen Homologen. Eine niedrige Geliertemperatur eines Weichmachers stellt eine positive Eigenschaft bei der Plastisolverarbeitung dar, da diese bei niedrigeren Temperaturen durchgeführt werden kann und zudem höhere Durchsätze bei der Verarbeitung erreicht werden können als bei der Verarbeitung von Plastisolen, welche Weichmacher mit einer hohen Geliertemperatur enthalten. Gleichzeitig weisen Terephthalsäureester mit geringem Molekulargewicht und dementsprechend geringer Kohlenstoffanzahl im Alkoholrest jedoch eine höhere Flüchtigkeit auf als ihre schwereren Homologen. Eine hohe Flüchtigkeit eines Weichmachers ist ein gravierender Nachteil, da mit Verlust des Weichmachers nicht nur die Eigenschaften des weichgemachten Polymers verändert werden und somit die Langlebigkeit des Produktes verringert wird, sondern auch Weichmacher in die Umgebung freigesetzt wird.

Diese Freisetzung von Weichmacher ist zudem problematisch, da beispielsweise auf den Gebieten der Innenraumanwendungen, medizinischen Produkte, Spielzeuge, Kabel und im Automobilsektor zur Kommerzialisierung der Produkte Normen erfüllt werden müssen, welche unter anderem die maximale Menge an austretenden organischen Verbindungen aus einem Produkt regeln um die für Verbraucher und Umwelt erforderliche Sicherheit zu gewährleisten. So regelt beispielsweise der Ausschuss zur gesundheitlichen Bewertung von Bauprodukten (AgBB) in Übereinstimmung mit der vom Europäischen Parlament verabschiedeten Bauprodukte-Verordnung (Nr. 305/2011) die Vermeidung und Begrenzung von Schadstoffen in Innenräumen. Demnach sind Bauprodukte und damit auch Weichmacher-haltige Produkte nur dann für die Verwendung in Innenräumen von Gebäuden aus gesundheitlicher Sicht geeignet, wenn bestimmte Grenzwerte für emittierte VOC (flüchtige organische Verbindungen) und SVOC (schwerflüchtige organische Verbindungen) in einem genormten Messverfahren nicht überschritten werden. In Anlehnung an die DIN ISO 16000-6 werden als SVOC solche organische Verbindungen eingestuft, welche auf einer unpolaren Säule im Retentionsbereich von > C16 - C22 liegen (AgBB - Bewertungsschema für VOC aus Bauprodukten, Stand 2012). Produkte, welche höhere Emissionen als zugelassen an VOC und/oder SVOC aufweisen, können nur dann eingesetzt werden, wenn zusätzliche Maßnahmen, wie z.B. das Aufbringen einer Emissions-Sperrschicht aus Lack, die Überschreitung der maximal erlaubten Emissionsmenge verhindern. Die Notwendigkeit solcher zusätzlichen Maßnahmen verringert jedoch die Freiräume bei der Konfektionierung der Weichmacher in Produkten und verteuert somit die jeweilige Anwendung von Weichmachern, welche als SVOC einzustufen sind. Zudem können sich durch die Notwendigkeit solcher zusätzlicher Schutzschichten weitere Schwierigkeiten ergeben wie beispielsweise eine erhöhte Anfälligkeit eines durch einen Lack geschützten SVOC-haltigen Produktes für Kratzer oder Abplatzungen.

Um die Eigenschaften des Weichmachersystems auf die Bedingungen der Verarbeitung und die geplante Anwendung einzustellen, werden oftmals Gemische von zwei oder mehr Weichmachern, beispielsweise zwei Terephthalsäurediestern mit unterschiedlichen Alkoholresten als Weichmacher für Polymere eingesetzt. Dementsprechend schlägt das Dokument US 2013/0317152 A1 vor, Di*iso*nonylterephthalat im Gemisch mit anderen Terephthalsäuredialkylestern, vorzugsweise solchen mit 4 bis 8 Kohlenstoffatomen im Alkoholrest, zu verwenden. Auch in Dokument US 2013/0310473 A1 werden Plastisole beschrieben, welche neben Dinonylterephthalat auch Di-n-butylterephthalat enthalten. Durch Einsatz von Mischungen mehrerer weichmachender Substanzen (auch Weichmacher genannt) zu einem Weichmachergemisch, welches oft vereinfacht ebenfalls als Weichmacher bezeichnet wird, ist es jedoch bislang nicht gelungen, sämtliche wünschenswerte Kombinationen von vorteilhaften Eigenschaften gezielt einzustellen. Beispielsweise offenbart das Dokument "Dibutyl Terephthalates in Plasticizer and related Applications" (IP.com Veröffentlichungsnummer: IPCOM000236730D, Veröffentlichungsdatum: 13.05.2014), dass Mischungen unterschiedlicher Terephthalsäureester Probleme bei der Verträglichkeit mit Polymeren aufwiesen. So zeigt in diesem Dokument die Abbildung 8, dass Dioctylterephthalat erst im Gemisch mit mehr als 50 % Dibutylterephthalat eine ausreichende Verträglichkeit mit Polymeren aufweist. Systeme umfassend Dioctylterephthalat und Dibutylterephthalat schwitzten demnach nur dann nicht aus dem Polymer aus, wenn sie mehr als 50 % Dibutylterephthalat enthielten, was den Variationsbereich der Gemischzusammensetzungen stark limitiert und damit auch die möglichweise erreichbaren Eigenschaften stark einschränkt.

Andere Dokumente schlagen vor, nicht nur unterschiedliche Terephthalsäurediester abzumischen, sondern die beiden Terephthalsäurediester zusammen mit dem zugehörigen Mischester als Weichmacher einzusetzen.

So legen die Dokumente KR 2013/0035493 A und US 2014/0336294 A1 das Problem dar, dass Dibutylterephthalat zwar eine hohe Geschwindigkeit bei dem Eindringen in das Harz und beim Schmelzen aufweise, jedoch einen unerwünscht hohen Migrationsverlust zeige, während bei Diethylhexylterephthalat kaum ein Migrationsverlust auftrete, das Eindringen in das Harz und das Schmelzen aber unakzeptabel lange dauere. Zur Verbesserung dieser gegenläufigen Eigenschaften schlagen diese Dokumente vor, Estergemische umfassend Mischester, welche einen C₄- und einen C₈-Alkoholrest enthalten, einzusetzen. Diese Estergemische weisen jedoch den Nachteil auf, dass sie Terephthalsäuredibutylester beinhalten, welche als SVOC-Komponente einzustufen sind und deren Einsatz dementsprechend den oben beschriebenen Einschränkungen bei der Formulierung unterliegt.

Das Dokument WO 2008/140177 A1 schlägt vor, Estergemische aus C₈- und C₉-Estern der Terephthalsäure herzustellen, welche wiederum auch den Mischester enthalten, und beschreibt, dass eine Verbesserung der Verarbeitbarkeit der Weichmacher-Zubereitungen durch die Variation der Verhältnisse der einzelnen Ester im Estergemisch erreicht werden könne.

Gegenüber diesen Terephthalsäureestergemischen der C₈- und C₉-Ester wiesen gemäß dem Dokument US 2014/0096703 A1 jedoch Terephthalsäureestergemische aus C₈- und C₁₀-Estern deutlich verbesserte Eigenschaften auf. Die in diesem Dokument vorgeschlagenen Systeme umfassen Mischester, welche einen C₈- und einen C₁₀-Alkoholrest enthalten, wiesen jedoch eine so schlechte Kompatibilität mit Polymeren auf, dass das Dokument selbst vorschlägt, zur Verbesserung der Kompatibilität dem Terephthalsäureestergemisch den Phthalat-Weichmacher Dipropylheptylphthalat zuzumischen.

Das Dokument US 2014/0336320 A1 schlägt neben Systemen, welche C₄- und C₈-Terephthalsäureester enthalten, ebenfalls solche mit C₈- und C₁₀-Alkoholresten vor.

In dem Dokument WO 2014/195055 A1 werden neben Terephthalsäureestergemischen mit C₈ und C₁₀-Alkoholresten auch solche mit C₇ und C₉-Alkoholresten beschrieben.

Der vorliegenden Erfindung lag nun die Aufgabe zugrunde, einige, vorzugsweise alle der oben genannten Nachteile des Standes der Technik zu überwinden. Es sollte vorzugsweise ein Weichmacher bereitgestellt werden, welcher zur Gewährleistung möglichst großer Freiräume bei der Formulierung nicht unter die Definitionen der nach deutschen oder internationalen Richtlinien reglementierten Verbindungen fällt. Dabei sollte mit Vorzug ein Weichmacher auf Basis von Terephthalsäureestern bereitgestellt werden, welcher gleichzeitig gute Eigenschaften im Bereich der Flüchtigkeit und der Geliertemperatur aufweist, also ein Weichmacher, der bei niedrigen Temperaturen geliert und gleichzeitig wenig flüchtig ist.

Diese Aufgabe wird gelöst durch ein Gemisch gemäß Anspruch 1.

Gegenstand der vorliegenden Erfindung ist deshalb ein Gemisch umfassend die Terephthalsäurediester I, II und III, 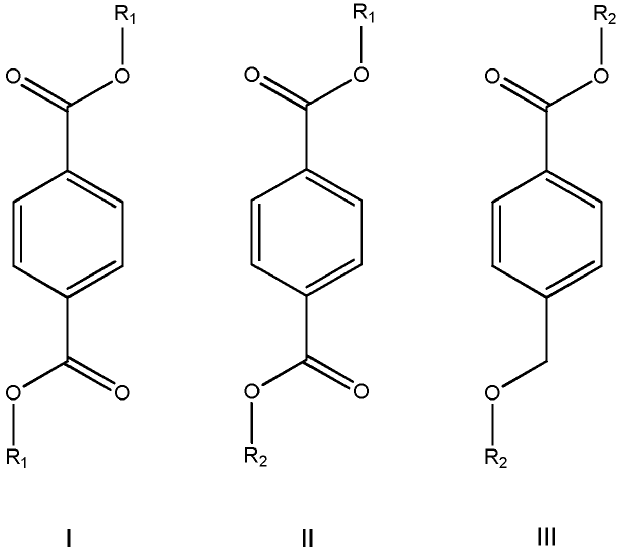 wobei R₁ für einen Alkylrest mit 5 Kohlenstoffatomen und R₂ für einen Alkylrest mit 9 Kohlenstoffatomen steht.

Im Rahmen dieser Anmeldung werden die Begriffe "R₁", "Alkylrest mit 5 Kohlenstoffatomen", "Pentyl-Rest" und "C₅-Rest" synonym verwendet. Selbiges gilt für die Begriffe "R₂", "Alkylrest mit 9 Kohlenstoffatomen", "Nonyl-Rest" und "C₉-Rest". Die Vorsilbe "*iso*" kennzeichnet, dass es sich um ein Isomerengemisch mit gemeinsamer Kohlenstoffanzahl handelt. Wird im Folgenden "ein Alkohol", beispielsweise "ein C₅-Alkohol" oder "ein Alkohol mit 5 Kohlenstoffatomen" beschrieben, so kann dieser nur aus einem einzigen Isomerem bestehen oder ein Gemisch mehrerer Isomere enthalten, also im Falle eines C₅-Alkohols ein *Iso*pentanol sein.

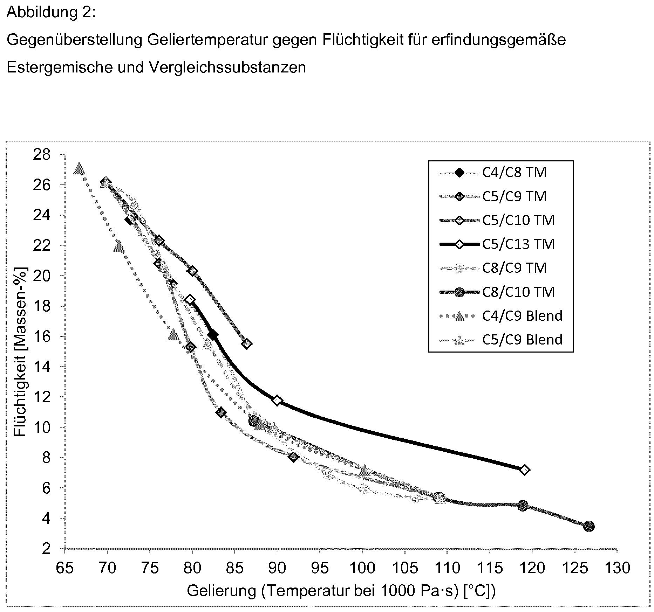

Überraschend wurde gefunden, dass erfindungsgemäße Gemische gegenüber bekannten Weichmacherkombinationen ein verbessertes Zusammenspiel der Eigenschaften Flüchtigkeit und Gelierungstemperatur aufweisen. Wie aus der Auftragung der Geliertemperatur gegen die Flüchtigkeit für die erfindungsgemäßen Gemische und andere Weichmachersysteme in der Abbildung 2 ersichtlich ist, weisen erfindungsgemäße Gemische im Vergleich zu den anderen Polymer-kompatiblen, SVOC-freien Weichmachersystemen niedrigere Geliertemperaturen und gleichzeitig niedrigere Flüchtigkeiten auf und zeigen somit gegenüber diesen Weichmachersystemen verbesserte Eigenschaften für Verarbeitung und Anwendung.

Vorzugsweise steht R₁ in den Formeln I, II und III für 2-Methylbutyl-, 3-Methylbutyl- und/oder *n*-Pentyl-Reste. Besonders bevorzugt steht R₁ in den Formeln I, II und III für *Iso*pentyl-Reste, wobei der Begriff *Iso*pentyl-Reste, wie oben definiert, ein Gemisch mehrerer isomerer Pentyl-Reste beschreibt. R₂ steht in den Formeln I, II und III bevorzugt für Nonyl- oder *n*-Nonyl-Reste. Besonders bevorzugt steht R₂ in den Formeln I, II oder III für *Iso*nonyl-Reste, wobei der Begriff *Iso*nonyl-Reste, wie oben definiert, ein Gemisch mehrerer isomerer Nonyl-Reste beschreibt.

Bevorzugt sind Gemische, in denen in den Formeln I, II und III R₁ für 2-Methylbutyl-, 3-Methylbutyl-, n-Pentyl- und/oder *Iso*pentyl-Reste steht und R₂ für *Iso*nonyl-Reste. Bevorzugt sind Gemische, in denen in den Formeln I, II und III R₂ für Nonyl-, *n*-Nonyl- und/oder *Iso*nonyl-Reste steht und R₁ für 2-Methylbutyl-Reste. Bevorzugt sind zudem Gemische, in denen in den Formeln I, II und III R₂ für Nonyl-, *n*-Nonyl- und/oder *Iso*nonyl-Reste steht und R₁ für 3-Methylbutyl-Reste. Bevorzugt sind des Weiteren Gemische, in denen in den Formeln I, II und III R₂ für Nonyl-, *n*-Nonyl- und/oder *Iso*nonyl-Reste steht und R₁ für n-Pentyl- und/oder *Iso*pentyl-Reste.

Vorzugsweise weisen in den Estern I, II und III des erfindungsgemäßen Gemisches enthaltene *Iso*nonyl-Reste einen durchschnittlichen Verzweigungsgrad von 1,0 bis 2,2 auf, da innerhalb dieses Bereiches die Verträglichkeit der erfindungsgemäßen Gemische mit Polymeren erhöht ist. Der durchschnittliche Verzweigungsgrad wird dabei bestimmt wie im Dokument US 2010/305255 A1 beschrieben.

In einer Ausführungsform liegt in dem erfindungsgemäßen Gemisch der Anteil der n-Pentyl-Reste bei mindestens 10 Mol-% oder 20 Mol-%, bevorzugt bei mindestens 30 Mol-%, dazu bevorzugt bei mindestens 40 Mol-%, besonders bevorzugt bei mindestens 50 Mol-% und insbesondere bei mindestens 60 Mol-%, basierend auf der Gesamtheit der in den Estern I, II und III enthaltenen Pentyl-Reste, was mit dem Vorteil einer geringeren und damit für die Verarbeitung von Plastisolen günstigeren Viskosität verknüpft sein kann. In einer bevorzugten Ausführungsform liegt der der Anteil der *n*-Pentyl-Reste basierend auf allen enthaltenen Pentyl-Resten zwischen 10 und 90 Mol-%, bevorzugt bei 20 bis 80 Mol-% und insbesondere bei 30 bis 70 Mol-%.

Eine besonders gute Verträglichkeit mit dem weichzumachenden Polymer wurde für solche erfindungsgemäßen Gemische gefunden, welche weniger als 80 Mol-%, bevorzugt weniger als 70 Mol-% und insbesondere weniger als 60 Mol-% des Esters III, bezogen auf die Gesamtheit der Ester I, II und III enthalten. Neben der verbesserten Verträglichkeit dieser Gemische gegenüber Gemischen, welche einen höheren Anteil an Ester III im Gemisch der Ester I, II und III enthalten, weisen Gemische mit weniger als 80 Mol-%, bevorzugt mit weniger als 70 Mol-% und insbesondere mit weniger als 60 Mol-% an Ester III zudem eine geringere und damit für die Verarbeitung von Plastisolen vorteilhaftere Geliertemperatur auf als Gemische, welche einen höheren Anteil an Ester III umfassen.

Eine geringere und damit verbesserte Flüchtigkeit weisen erfindungsgemäße Gemische auf, welche weniger als 60 Mol-%, vorzugsweise weniger als 50 Mol-%, weiter bevorzugt weniger als 40 Mol-%, ganz besonders bevorzugt weniger als 30 Mol-% und insbesondere weniger als 20 Mol-% des Esters I, bezogen auf die Gesamtheit der Ester I, II und III, enthalten, weshalb solche Gemische ebenfalls bevorzugt sind.

Erfindungsgemäße Gemische mit besonders günstiger Geliertemperatur liegen vor, wenn das Gemisch vorzugsweise weniger als 40 Mol-% und insbesondere weniger als 30 Mol-% des Esters III, bezogen auf die Gesamtheit der Ester I, II und III, enthält. Besonders wenig flüchtige Gemische umfassen vorzugsweise mehr als 30 Mol-% und insbesondere mehr als 40 Mol-% des Esters III, bezogen auf die Gesamtheit der Ester I, II und III.

In einer Ausführungsform der vorliegenden Erfindung umfasst das Gemisch mindestens 10 Mol-%, bevorzugt mindestens 20 Mol-%, besonders bevorzugt mindestens 30 Mol-% und insbesondere mindestens 40 Mol-% des Esters II und/oder maximal 60 Mol-%, bevorzugt maximal 50 Mol-%, besonders bevorzugt maximal 40 Mol-% und insbesondere maximal 30 Mol-% des Esters II, jeweils bezogen auf die Gesamtheit der Ester I, II und III.

Die erfindungsgemäßen Gemische umfassen vorzugsweise weniger als 40 Gew.-%, weiter bevorzugt weniger als 30 Gew.-%, dazu bevorzugt weniger als 20 Gew.-% und insbesondere weniger als 10 Gew.-% an Komponenten, welche nicht unter die Definition der Ester I, II und III fallen. Insbesondere enthalten erfindungsgemäße Gemische mit Vorzug weniger als 20 Mol-%, bevorzugt weniger als 10 Mol-% und insbesondere weniger als 3 Mol-% an Terephthalsäurediestern, welche nicht unter die Definition der Ester I, II und III fallen, wobei bei der Bestimmung der Molanteile die Gesamtheit aller Terephthalsäurediester im Gemisch berücksichtigt wird.

Ein bevorzugter Gegenstand der vorliegenden Erfindung ist ein Gemisch umfassend die Terephthalsäurediester I, II und III, 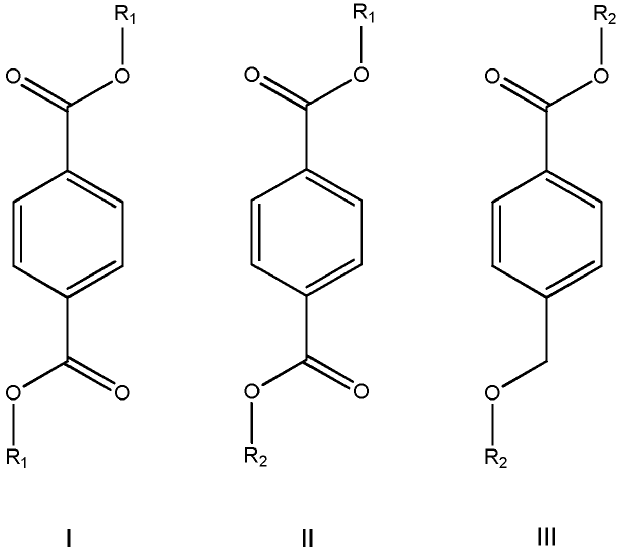 wobei R₁ für 2-Methylbutyl-, 3-Methylbutyl-, n-Pentyl- und/oder *Iso*pentyl-Reste und R₂ für *Iso*nonyl-Reste steht und welches weniger als 80 Mol-%, bevorzugt weniger als 70 Mol-% und insbesondere weniger als 60 Mol-% des Esters III, bezogen auf die Gesamtheit der Ester I, II und III enthält.

Ein weiterer Gegenstand der vorliegenden Erfindung ist die Verwendung eines erfindungsgemäßen Gemisches als Weichmacher für Polymere. Hierbei ist der Begriff Weichmacher - wie oben bereits erläutert - so zu verstehen, dass das erfindungsgemäße Estergemisch allein der Weichmacher ist, oder das erfindungsgemäße Gemisch zusammen mit weiteren Polymer-weichmachenden Komponenten den Weichmacher bildet.

Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und CelluloseDerivate, insbesondere Nitrocellulose (NC), Ethylcellulose (EC), Celluloseacetat (CA), Cellulose-Acetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Besonders bevorzugt ist die Verwendung eines erfindungsgemäßen Estergemisches als Weichmacher für PVC.

Vorzugsweise wird das Estergemisch als Weichmacher in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, verwendet.

Weiterer Gegenstand der vorliegenden Erfindung ist ein Mittel enthaltend ein erfindungsgemäßes Estergemisch, sowie ein oder mehrere Polymere aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Bezogen auf 100 Massenteile Polymer enthalten bevorzugte Mittel von 5 bis 200, bevorzugt von 10 bis 150 Massenteile an Weichmacher.

Bevorzugt ist die Verwendung des erfindungsgemäßen Estergemisches als Weichmacher für Polyvinylchlorid und dementsprechend sind Mittel, welche das erfindungsgemäße Estergemisch und PVC enthalten, besonders bevorzugt.

Bevorzugt ist das Polymer ein Suspensions-, Masse-, Mikrosuspensions- oder Emulsions-PVC.

Bevorzugte erfindungsgemäße Mittel können neben dem erfindungsgemäßen Estergemisch mindestens eine weitere Polymer-weichmachende Verbindung, also einen weiteren Weichmacher, enthalten. Eine besonders bevorzugte Ausführungsform des erfindungsgemäßen Mittels liegt vor, wenn dieses weniger als 5 Massen-% und insbesondere weniger als 0,5 Massen-% Phthalat-haltige Verbindungen enthält. Die weiteren Weichmacher werden vorzugsweise ausgewählt aus der Gruppe der Adipate, Benzoate, beispielsweise Monobenzoate oder Glycoldibenzoate, chlorierten Kohlenwasserstoffe, Citrate, Cyclohexandicarboxylate, epoxidierten Fettsäureester, epoxidierten Pflanzenöle, epoxidierten acylierten Glyceride, Furandicarboxylate, Phosphate, Phthalate (vorzugsweise in möglichst geringen Mengen), Succinate, Sulfonamide, Sulfonate, Terephthalate, Trimellitate oder oligomeren oder polymeren Ester auf Basis von Adipin-, Bernstein- oder Sebacinsäure. Besonders bevorzugt sind Alkylbenzoate, Dialkyladipate, Glycerinester, Citronensäuretrialkylester, acylierte Citronensäuretrialkylester, Trialkyltrimellitate, Glykoldibenzoate, Dialkylterephthalate, Ester der Furandicarbonsäure, Dialkanoylester von Dianhydrohexitolen (z.B. Isosorbit) und Dialkylester der 1,2-, 1,3- oder 1,4-Cyclohexandicarbonsäure.

In einer Ausführungsform enthält das erfindungsgemäße Mittel neben dem erfindungsgemäßen Estergemisch weniger als 20 Massen-%, weniger als 10 Massen-% oder keinen weiteren Weichmacher, wobei die Massen-% auf der Gesamtmasse des Mittels basieren.

Erfindungsgemäße Mittel enthalten vorzugsweise neben dem Polymer oder einer Mischung mehrerer Polymere und dem erfindungsgemäßen Estergemisch ein oder mehrere Additive aus der Gruppe der Thermostabilisatoren, Füllstoffe, Pigmente, Treibmittel, Biozide, UV- und Licht-Stabilisatoren, Co-Stabilisatoren, Antioxidantien, Viskositätsregler, Entlüfter, Haftvermittler, Gleitmittel und Färbemittel.

Die erfindungsgemäßen Mittel können in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, eingesetzt werden.

Erfindungsgemäße Gemische können beispielsweise durch Veresterungs- oder Umesterungsverfahren hergestellt werden.

### Herstellungsverfahren I

Eine Ausführungsform der vorliegenden Erfindung besteht darin, erfindungsgemäße Gemische mittels Veresterung von Terephthalsäure oder einem Terephthalsäurederivat mit einem Alkoholgemisch von C₅- und C₉-Alkoholen herzustellen.

Gegenstand der vorliegenden Erfindung ist ein Verfahren zur Herstellung eines erfindungsgemäßen Estergemisches durch Verestern von Terephthalsäure oder von einem Terephthalsäurederivat mit einem Gemisch umfassend R₁OH (R₁= Alkylrest mit 5 Kohlenstoffatomen) und R₂OH (R₂= Alkylrest mit 9 Kohlenstoffatomen). Ein bevorzugte eingesetztes Terephthalsäurederivat ist Dimethylterephthalat (DMT).

### Herstellungsverfahren II

In einer anderen Ausführungsform der vorliegenden Erfindung wird das erfindungsgemäße Gemisch hergestellt, indem der Ester I mit weniger als 1 Mol-Äquivalent, bezogen auf die Anzahl seiner Esterfunktionen, an Alkohol umgesetzt wird, welcher 9 Kohlenstoffatome enthält.

Gegenstand der vorliegenden Erfindung ist ein Verfahren zur Herstellung eines erfindungsgemäßen Estergemisches, in welchem der Ester Imit weniger als 1 Mol-Äquivalent, bezogen auf die Anzahl seiner Esterfunktionen, eines Alkohols oder mehrerer Alkohole mit 9 Kohlenstoffatomen (kurz: C₉-Alkohol) umgesetzt und dabei (optional in Gegenwart eines Katalysators) vorzugsweise zum Sieden erhitzt wird.

Die Reste R₁ und R₂ sind dabei wie weiter oben für die erfindungsgemäßen Gemische definiert. Besonders bevorzugt steht R₁ für 2-Methylbutyl-, 3-Methylbutyl-, n-Pentyl- und/oder *Iso*pentyl-Reste und R₂ für *Iso*nonyl-Reste, wobei das Gemisch vorzugsweise weniger als 80 Mol-%, bevorzugt weniger als 70 Mol-% und insbesondere weniger als 60 Mol-% des Esters III, bezogen auf die Gesamtheit der Ester I, II und III, enthält. Es gelten auch die weiter oben angegebenen bevorzugten Ausführungsformen hinsichtlich der Isomerenverteilungen von R₁ und R₂.

In diesem Herstellungsverfahren II wird der Ester Imit weniger C₉-Alkohol umgesetzt, als zum vollständigen Austausch sämtlicher Alkoholreste der Esterfunktionen innerhalb des Esters Inotwendig wäre. Überraschenderweise wurde festgestellt, dass dann die Zusammensetzung des resultierenden Estergemisches umfassend die ungemischten Ester Iund III sowie den Mischester II gezielt - im Rahmen der sich für den Fall des vollständigen Einbaus der Alkoholreste des C₉-Alkohols ergebenden Statistik - eingestellt werden kann, wenn der Ester Ivorgelegt und der C₉-Alkohol zugesetzt wird. Eine Steuerung der Estergemisch-Zusammensetzung ist hingegen nicht möglich, wenn in einer Veresterung oder Umesterung ein Alkoholgemisch (C₅-Alkohol + C₉-Alkohol) der einzuführenden Alkoholreste eingesetzt wird oder zu dem Ester III C₅-Alkohol zugesetzt wird.

So ist es mittels des Herstellungsverfahrens II möglich, Estergemische bereitzustellen, in denen die unterschiedlichen Alkoholreste in einer vorbestimmten Mengenverteilung enthalten sind und in denen zudem die Mengenverteilung der enthaltenen Ester gezielt - im Rahmen der oben genannten Statistik - gesteuert werden kann. Somit ist es möglich, Estergemische bereitzustellen, deren Zusammensetzung geringere Abweichungen von der sich aufgrund der Statistik ergebenden Verteilung der Ester aufweist, als Estergemische, welche nach im Stand der Technik beschriebenen, ungesteuerten Verfahren hergestellt werden.

Für die mittels des Herstellungsverfahrens II hergestellten erfindungsgemäßen Gemische ergeben sich die in der Tabelle 1 dargestellten statistischen Erwartungswerte.

**Tabelle 1:statistische Erwartungswerte (Herstellungsverfahren II)**

| Einsatz | | Erwartungsmenge | | |
|---|---|---|---|---|
| Ester I [C₅-Äquivalente] | C₉-Alkohol [C₉-Äquivalente] | Ester I [Mol-%] | Ester II [Mol-%] | Ester III [Mol-%] |
| 2 | 0,2 | 81 | 18 | 1 |
| 2 | 0,4 | 64 | 32 | 4 |
| 2 | 0,6 | 49 | 42 | 9 |
| 2 | 0,8 | 36 | 48 | 16 |
| 2 | 1,0 | 25 | 50 | 25 |
| 2 | 1,2 | 16 | 48 | 36 |
| 2 | 1,4 | 9 | 42 | 49 |
| 2 | 1,6 | 4 | 32 | 64 |
| 2 | 1,8 | 1 | 18 | 81 |

Eine Quantifizierung der Abweichung der Estergemischzusammensetzung von der aus statistischen Überlegungen resultierenden Mengenverteilung der Ester im Estergemisch ist durch Summenbildung aller Beträge der Differenzen zwischen dem statistischem Erwartungswert, welcher sich ergibt, wenn man von einem vollständigen Einbau der Alkoholreste des C₉-Alkohols ausgeht, und dem tatsächlichen Mol-Anteil jedes einzelnen Esters im Estergemisch für den Fall, dass sich die Summe der Mol-Anteile der oben genannten Ester im Estergemisch zu 100 addiert, möglich.

Mittels des Herstellungsverfahrens II gelingt es beispielsweise aus 5,5 Mol Di*iso*pentylterephthalat und 7,1 Mol *Iso*nonanol gezielt ein Estergemisch bereitzustellen, in dem die Molverteilung der enthaltenen Ester lediglich um 4 Punkte vom statistischen Erwartungswert abweicht und der Anteil der *Iso*pentyl-Reste im Estergemisch bis auf 1 % dem anvisierten *Iso*pentylanteil entspricht.

Erfindungsgemäß wird in dem Herstellungsverfahren II weniger als 1 Mol-Äquivalent bezogen auf die Anzahl im Ester Ienthaltener Esterfunktionen (1 Mol-Äquivalent C₅-Äquivalente) an C₉-Alkohol eingesetzt. Bevorzugt wird der Ester I mit weniger als 0,98 Mol-Äquivalent, bevorzugt mit weniger als 0,95 Mol-Äquivalent und insbesondere mit weniger als 0,90 Mol-Äquivalent, bezogen auf die Anzahl seiner Esterfunktionen, des C₉-Alkohols umgesetzt. Es kann auch bevorzugt sein, den Ester I mit weniger als 0,85 Mol-Äquivalent, bevorzugt mit weniger als 0,80 Mol-Äquivalent und insbesondere mit weniger als 0,75 Mol-Äquivalent, bezogen auf die Anzahl seiner Esterfunktionen, des C₉-Alkohols umzusetzen. Auf diese Weise werden Estergemische erhalten, welche gegenüber dem Ester III deutlich günstigere, das heißt niedrigere, Geliertemperaturen aufweisen und sich gleichzeitig gegenüber dem Ester I durch eine geringere und damit verbesserte Flüchtigkeit auszeichnen. Estergemische, welche besonders gut mit Polymeren, beispielsweise PVC, verarbeitbar sind, werden erhalten, wenn der Ester I mit weniger als 0,85 Mol-Äquivalent, bevorzugt mit weniger als 0,80 Mol-Äquivalent und insbesondere mit weniger als 0,75 Mol-Äquivalent, bezogen auf die Anzahl seiner Esterfunktionen, C₉-Alkohol umgesetzt wird.

Mit Vorzug wird der Ester I mit mehr als 0,05 Mol-Äquivalent, bevorzugt mit mehr als 0,10 Mol-Äquivalent und insbesondere mit mehr als 0,20 Mol-Äquivalent, bezogen auf die Anzahl seiner Esterfunktionen, des C₉-Alkohols umgesetzt. In vielen Fällen ist es von Vorzug, den Ester I mit mehr als 0,25 Mol-Äquivalent, bevorzugt mit mehr als 0,30 Mol-Äquivalent und insbesondere mit mehr als 0,35 Mol-Äquivalent bezogen auf die Anzahl seiner Esterfunktionen, des C₉-Alkohols umzusetzen. Hierdurch können Estergemische erhalten werden, welche zur Herstellung von Estergemisch- und Polymer-haltigen Mitteln mit besonders guter Lagerfähigkeit geeignet sind. Eine weitere Verbesserung der Eigenschaften im Bereich der Flüchtigkeit wird möglich, wenn der Ester I mit mehr als 0,70 Mol-Äquivalent, bevorzugt mit mehr als 0,75 Mol-Äquivalent und insbesondere mit mehr als 0,80 Mol-Äquivalent, bezogen auf die Anzahl seiner Esterfunktionen, des C₉-Alkohols umgesetzt wird.

Ein bevorzugter Gegenstand der vorliegenden Erfindung ist ein Verfahren zur Herstellung eines erfindungsgemäßen Estergemisches umfassend die Ester Dipentylterephthalat, Di*iso*nonylterephthalat und Pentyl(*iso*nonyl)terephthalat, in welchem Dipentylterephthalat mit weniger als 1 Mol-Äquivalent, bezogen auf die Anzahl seiner Esterfunktionen, *Iso*nonanol umgesetzt und dabei zum Sieden erhitzt wird. Hierbei wird Dipentylterephthalat bevorzugt mit mehr als 0,05 Mol-Äquivalent aber weniger als 0,98 Mol-Äquivalent, mit weniger als 0,95 Mol-Äquivalent, mit weniger als 0,90 Mol-Äquivalent, mit weniger als 0,85 Mol-Äquivalent, mit weniger als 0,80 Mol-Äquivalent, mit weniger als 0,75 Mol-Äquivalent, mit weniger als 0,70 Mol-Äquivalent oder mit weniger als 0,65 Mol-Äquivalent, bezogen auf die Anzahl seiner Esterfunktionen, des Alkohols *Iso*nonanol umgesetzt und dabei zum Sieden erhitzt. Bevorzugt weisen dabei die Pentyl-Reste und die *Iso*nonyl-Reste die weiter oben für die erfindungsgemäßen Gemische erläuterten Isomerenzusammensetzungen auf.

Zur Verbesserung der Steuerbarkeit der Zusammensetzung des aus der Umsetzung des Herstellungsverfahrens II resultierenden Estergemisches enthält die Reaktionsmischung während der Umsetzung mit Vorzug weniger als 0,5 Mol-Äquivalent, bevorzugt weniger als 0,1 Mol-Äquivalent, besonders bevorzugt weniger als 0,05 Mol-Äquivalent und insbesondere weniger als 0,01 Mol-Äquivalent an Alkoholen, welche keine C₅- und keine C₉-Alkohole sind, wobei sich die Mol-Äquivalente auf die Gesamtheit aller in der Reaktionsmischung enthaltenen Alkohole (entsprechend 1 Mol-Äquivalent) beziehen.

Die gewünschte Zusammensetzung des herzustellenden Estergemisches kann besonders gut gesteuert und das Verfahrensprodukt nach einem geringeren Aufarbeitungsaufwand direkt als Weichmacher oder Weichmacherkomponente genutzt werden, wenn die in dem erfindungsgemäßen Verfahren eingesetzten Komponenten umfassend Ester I und C₉-Alkohol weniger als 50 Vol.-%, vorzugsweise weniger als 35 Vol.-% und insbesondere weniger als 20 Vol.-%, weiter bevorzugt weniger als 10 Vol.-% an Komponenten enthält, welche keine Edukte, End- oder Zwischenprodukte der Umsetzung von Ester I mit C₉-Alkohol sind. Insbesondere sollten die in dem Verfahren eingesetzten Komponenten umfassend Ester I und C₉-Alkohol vorzugsweise weniger als 15 Gew.-%, bevorzugt dazu weniger als 10 Gew.-% und insbesondere weniger als 5 Gew.-% Säuregruppen-haltige Terephthalsäurederivate enthalten, um die Steuerbarkeit der Umesterung nicht zu gefährden.

Als Lösungsmittel kann im Herstellungsverfahren II beispielsweise Toluol, Xylol, Benzol, Cyclohexan oder C₅-Alkohol, vorzugsweise in Mengen bis zu maximal 50 Vol-%, bevorzugt bis zu maximal 35 Vol-% und insbesondere bis zu maximal 20 Vol-%, jeweils bezogen auf das gesamte Reaktionsgemisch, eingesetzt werden. Vorzugsweise liegt der Anteil der Lösungsmittel, welche kein C₅-Alkohol sind, jedoch unterhalb von 30 Vol-%, vorzugsweise unterhalb von 15 Vol-% und insbesondere unterhalb von 1 Vol-%, jeweils bezogen auf das gesamte Reaktionsgemisch.

In bevorzugten Ausführungsformen ermöglicht das Herstellungsverfahren II die Bereitstellung von erfindungsgemäßen Gemischen, in welchem das Molverhältnis der Ester I, II und III um weniger als 15 Punkte, vorzugsweise um weniger als 10 Punkte vom statistisch ermittelten Erwartungswert abweicht, welcher sich ergibt, wenn man von einem vollständigen Einbau der C₉-Alkoholreste ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Das Herstellungsverfahren II und das nachfolgend noch erläuterte Herstellungsverfahren III werden vorzugsweise in Anwesenheit eines Katalysators oder mehrerer Katalysatoren, beispielsweise unter Verwendung von Brönstedt- oder Lewis-Säuren oder -Basen als Katalysator, durchgeführt. Als besonders geeignete Katalysatoren haben sich Schwefelsäure, Methansulfonsäure, *p*-Toluolsulfonsäure, Metalle oder deren Verbindungen erwiesen. Beispiele für besonders bevorzugte Metallkatalysatoren sind Zinnpulver, Zinn(II)oxid, Zinn(II)oxalat, Titansäureester wie Tetra*iso*propylorthotitanat oder Tetrabutylorthotitanat sowie Zirkoniumester wie Tetrabutylzirkonat sowie Natriummethanolat und Kaliummethanolat.

Das Herstellungsverfahren II kann in dem Fachmann bekannten, typischen Veresterungsapparaturen unter üblichen Verfahrensbedingungen durchgeführt werden. Vorzugsweise findet das Verfahren bei Temperaturen bei oder oberhalb des Siedepunktes des C₅-Alkohols statt, so dass der bei der Reaktion gebildete C₅-Alkohol bei dem vorgegebenen Druck aus dem Reaktionsgemisch abdestilliert werden kann. Zur Gewährleistung einer quantitativen Rückführung in der Gasphase befindlichen C₉-Alkohols, sollte die Veresterungsapparatur vorzugsweise mit einer Kolonne ausgestattet sein. Unter dem Begriff "quantitativ" wird in diesem Zusammenhang zu mehr als 80 Mol-%, vorzugsweise zu mehr als 90 Mol-% und insbesondere zu mehr als 95 Mol-%, basierend auf der Menge des eingesetzten C₉-Alkohols, verstanden.

In einer besonders bevorzugten Ausführungsform des Verfahrens werden mehr als 50 Mol-%, vorzugsweise mehr als 60 Mol-%, bevorzugt mehr als 70 Mol-%, besonders bevorzugt mehr als 80 Mol-%, mit Vorzug mehr als 90 Mol-%, mit besonderem Vorzug mehr als 95 Mol-% und insbesondere mehr als 99 Mol-% des sich im Laufe des Verfahrens bildenden C₅-Alkohols während des Verfahrens - vorzugsweise mittels Destillation - aus dem Reaktionsgefäß entfernt.

Vorzugsweise werden während der Reaktion in regelmäßigen Abständen GC-Chromatogramme angefertigt um den Fortschritt der Reaktion zu beobachten. Mit Vorzug wird die Reaktion durch Abkühlen und/oder Zerstören des Katalysators, beispielsweise durch Zugabe von Wasser und/oder Base, abgebrochen, nachdem in den GC-Chromatogrammen ein Restgehalt an eingesetztem C₉-Alkohol von kleiner als 5,0 Flächen-%, bevorzugt von kleiner als 2 Flächen-%, besonders bevorzugt kleiner 1,0 Flächen-% und insbesondere von kleiner 0,5 Flächen-%, bezogen auf die Gesamtfläche aller Ester im GC-Chromatogramm, festgestellt wird.

Es ist besonders bevorzugt, dass der Katalysator zerstört wird, nachdem mehr als 60 Mol-%, bevorzugt mehr als 70 Mol-%, besonders bevorzugt mehr als 80 Mol-%, mit Vorzug mehr als 90 Mol-% und insbesondere mehr als 95 Mol-% des sich im Laufe des Verfahrens bildenden C₅-Alkohols aus dem Reaktionsgefäß entfernt wurden. Hierbei findet die Zerstörung des Katalysators vorzugsweise statt, nachdem mittels Reaktionskontrolle ein Reaktionsfortschritt von mindestens 90 % festgestellt wurde, beispielsweise indem in den Reaktionsfortschritt kontrollierenden GC-Chromatogrammen ein Restgehalt an eingesetztem C₉-Alkohol von kleiner als 5,0 Flächen-%, bevorzugt von kleiner als 2,0 Flächen-% und insbesondere von kleiner 1,0 Flächen-%, bezogen auf die Gesamtfläche aller Ester im GC-Chromatogramm, festgestellt wird.

Nach Beendigung der Reaktion wird das Reaktionsgemisch in üblicher Art und Weise aufgearbeitet.

Bevorzugter Gegenstand der vorliegenden Erfindung ist ein Verfahren zur Herstellung eines erfindungsgemäßen Estergemisches umfassend die Ester I, II und III, in welchem der Ester I mit weniger als 1 Mol-Äquivalent, bezogen auf die Anzahl seiner Esterfunktionen, mit einem Alkohol oder mehreren Alkoholen mit 9 Kohlenstoffatomen - vorzugsweise in Anwesenheit eines Katalysators - umgesetzt und dabei zum Sieden erhitzt wird, wobei mehr als 80 Mol-%, vorzugsweise mehr als 90 Mol-% und insbesondere mehr als 95 Mol-% des sich im Laufe des Verfahrens bildenden C₅-Alkohols während des Verfahrens - vorzugsweise mittels Destillation - aus dem Reaktionsgefäß entfernt werden.

Hierbei weicht vorzugsweise das Molverhältnis der Ester I, II und III um weniger als 15 Punkte, vorzugsweise um weniger als 10 Punkte von dem statistisch ermittelten Erwartungswert ab, welcher sich ergibt, wenn man von einem vollständigen Einbau der C₉-Alkoholreste ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

### Herstellungsverfahren III

In einer anderen Ausführungsform der vorliegenden Erfindung wird das erfindungsgemäße Gemisch hergestellt, indem Terephthalsäure oder ein Terephthalsäurederivat mit einem Gemisch von C₅- und C₉-Alkoholen umgesetzt wird, wobei der C₅-Alkohol im Überschuss, der C₉-Alkohol jedoch in der Menge eingesetzt wird, die der Menge der C₉-Estergruppen in dem herzustellenden erfindungsgemäßen Estergemisch entspricht.

Gegenstand der vorliegenden Erfindung ist ein Verfahren zur Herstellung eines erfindungsgemäßen Estergemisches umfassend die Terephthalsäureester I, II und III durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als der Alkohol R₁OH des Restes R₁ bei demselben Druck, mit einer Menge (m₁+ s₁) R₁OH und einer Menge m₂ R₂OH, wobei das Reaktionsgemisch zum Sieden erhitzt wird und
- m₁ und m₂: den Mol-Äquivalenten der in Terephthalsäure oder deren Derivaten einzuführenden Alkoholresten OR₁ (C₅-Alkoholrest) und OR₂ (C₉-Alkoholrest) entspricht und
- s₁: größer als 0 ist.

Vorzugsweise wird in dem Herstellungsverfahren III das Terephthalsäurederivat Dimethylterephthalat (DMT) eingesetzt.

Wie bereits für die erfindungsgemäßen Gemische beschrieben, steht R₁ dabei für 2-Methylbutyl-, 3-Methylbutyl- und/oder *n*-Pentyl-Reste. Besonders bevorzugt steht R₁ in den Formeln I, II und III für *Iso*pentyl-Reste, wobei der Begriff *Iso*pentyl-Reste, wie oben definiert, ein Gemisch mehrerer isomerer Pentyl-Reste beschreibt. R₂ steht vorzugsweise für Nonyl-, *n*-Nonyl-Reste, oder ein Gemisch mehrerer isomerer Nonyl-Reste, *Iso*nonyl-Reste genannt. Hierbei weisen dabei die Pentyl-Reste und die *Iso*nonyl-Reste bevorzugt zudem die weiter oben für die erfindungsgemäßen Gemische erläuterten Isomerenzusammensetzungen auf. Besonders bevorzugt steht R₁ für 2-Methylbutyl-, 3-Methylbutyl-, n-Pentyl- und/oder *Iso*pentyl-Reste und R₂ für *Iso*nonyl-Reste, wobei das Gemisch vorzugsweise weniger als 80 Mol-%, bevorzugt weniger als 70 Mol-% und insbesondere weniger als 60 Mol-% des Esters III, bezogen auf die Gesamtheit der Ester I, II und III, enthält.

In einer Ausführungsform wird in diesem Herstellungsverfahren III Terephthalsäure zu den Estern I, II und III verestert.

In einer anderen Ausführungsform wird in diesem Herstellungsverfahren III ein Terephthalsäurediester zu den Estern I, II und III umgeestert. Hierbei weist der Alkohol ROH des Diesters einen niedrigeren Siedepunkt bei einem bestimmten Druck auf als der/die C₅-Alkohol(e) bei demselben Druck.

Mit Vorzug werden die im Zuge des Herstellungsverfahrens III in Terephthalsäure oder deren Derivaten eingeführten Alkoholreste zu mindestens 95 Mol-%, bevorzugt zu mindestens 98 Mol-%, besonders bevorzugt zu mindestens 99 Mol-% und insbesondere zu 100 Mol-% Teil einer Esterfunktion.

In dem Herstellungsverfahren III wird Terephthalsäure oder ein Derivat davon mit einem Gemisch von C₅- und C₉-Alkoholen umgesetzt. Überraschenderweise wurde festgestellt, dass dann die Zusammensetzung des resultierenden Estergemisches umfassend die ungemischten Ester Iund III sowie der Mischester II gezielt - im Rahmen der sich für den Fall des vollständigen Einbaus der C₉-Alkoholreste ergebenden Statistik - eingestellt werden kann, wenn der C₅-Alkohol im Überschuss zu den in Terephthalsäure oder deren Derivaten einzuführenden C₅-Alkoholresten eingesetzt wird, die Menge des eingesetzten C₉-Alkohols jedoch der Menge der einzuführenden C₉-Alkoholreste entspricht. Eine Steuerung der Estergemisch-Zusammensetzung ist hingegen nicht möglich, wenn in einer Veresterung oder Umesterung ein Überschuss des Gemisches der C₅- und C₉-Alkohole in Bezug auf die einzuführenden Alkoholreste eingesetzt wird oder Terephthalsäure oder ein Derivat davon mit einem Gemisch der C₅- und C₉-Alkohole umgesetzt wird, wobei der C₉-Alkohol zu den in der Terephthalsäure oder deren Derivaten einzuführenden C₉-Alkoholresten im Überschuss eingesetzt wird.

So ist es mittels des Herstellungsverfahrens III möglich, Estergemische bereitzustellen, in denen die unterschiedlichen Alkoholreste in einer vorbestimmten Mengenverteilung enthalten sind und in denen zudem die Mengenverteilung der enthaltenen Ester gezielt - im Rahmen der oben genannten Statistik - gesteuert werden kann. Somit ist es möglich, Estergemische bereitzustellen, deren Zusammensetzung geringere Abweichungen von der sich aufgrund der Statistik ergebenden Verteilung der Ester aufweist, als Estergemische, welche nach im Stand der Technik beschriebenen, ungesteuerten Verfahren hergestellt werden.

Für x = 1 ergeben sich die in der Tabelle 1 dargestellten statistischen Erwartungswerte der Estergemisch-Zusammensetzung.

**Tabelle 2: statistische Erwartungswerte (Herstellungsverfahren III)**

| Einsatz - Verhältnis C₅OH zu C₉OH (m₁ : m₂) | | Erwartungsmenge | | |
|---|---|---|---|---|
| m₁ | m₂ | Ester I [Mol-%] | Ester II [Mol-%] | Ester III [Mol-%] |
| 9 | 1 | 81 | 18 | 1 |
| 8 | 2 | 64 | 32 | 4 |
| 7 | 3 | 49 | 42 | 9 |
| 6 | 4 | 36 | 48 | 16 |
| 5 | 5 | 25 | 50 | 25 |
| 4 | 6 | 16 | 48 | 36 |
| 3 | 7 | 9 | 42 | 49 |
| 2 | 8 | 4 | 32 | 64 |
| 1 | 9 | 1 | 18 | 81 |

Eine Quantifizierung der Abweichung der Estergemischzusammensetzung von der aus statistischen Überlegungen resultierenden Mengenverteilung der Ester im Estergemisch ist durch Summenbildung aller Beträge der Differenzen zwischen dem statistischem Erwartungswert, welcher sich ergibt, wenn man von einem vollständigen Einbau der C₉-Alkoholreste ausgeht, und dem tatsächlichen Mol-Anteil jedes einzelnen Esters im Estergemisch für den Fall, dass sich die Summe der Mol-Anteile der oben genannten Ester im Estergemisch zu 100 addiert, möglich.

Mittels des erfindungsgemäßen Verfahrens gelingt es beispielsweise aus 5 Mol Dimethylterephthalat, 3,5 Mol *Iso*pentanol und 9 Mol *Iso*nonanol gezielt ein Estergemisch bereitzustellen, in denen die Molverteilung der enthaltenen Ester lediglich um 1 Punkt vom statistischen Erwartungswert abweicht und der Anteil der Pentyl-Reste im Estergemisch bis auf 0,3 % dem anvisierten Pentylanteil entspricht.

Mit Vorzug werden in dem Herstellungsverfahren III die C₅- und C₉-Alkohole in einem solchen Verhältnis zueinander eingesetzt, dass das Verhältnis von m₁ zu m₂ (m₁:m₂) im Bereich von 1:9 bis 9:1, vorzugsweise im Bereich von 2:8 bis 8:2, besonders bevorzugt im Bereich von 3:7 bis 7:3 und insbesondere im Bereich von 4:6 bis 6:4 liegt. Werden die C₅-und C₉-Alkohole in einem Verhältnis m₁:m₂ größer 1,5:8,5, vorzugsweise größer 2:8 und insbesondere größer 2,5:7,5 eingesetzt, können Estergemische erhalten werden, welche gegenüber dem Ester III deutlich günstigere, das heißt niedrigere, Geliertemperaturen aufweisen und sich gleichzeitig gegenüber dem Ester Idurch eine geringere und damit verbesserte Flüchtigkeit auszeichnen. Estergemische, welche besonders gut mit Polymeren, beispielsweise PVC verarbeitet werden können, können erhalten werden, wenn die C₅- und C₉-Alkohole im einem Verhältnis m₁:m₂ eingesetzt werden, welches größer ist als 1,5:8,5, bevorzugt größer ist als 2:8 und insbesondere größer ist als 2,5:7,5. Estergemische, welche sich zur Herstellung von Estergemisch- und Polymer-haltigen Mitteln mit guter Lagerfähigkeit eignen, können erhalten werden, wenn die C₅- und C₉-Alkohole in einem Verhältnis m₁:m₂ kleiner 8:2, vorzugsweise kleiner 7,5:2,5 und insbesondere kleiner 7:3 eingesetzt werden. Eine weitere Verbesserung der Eigenschaften im Bereich der Flüchtigkeit wird möglich, wenn die C₅- und C₉-Alkohole in einem Verhältnis m₁:m₂ kleiner 4:6, vorzugsweise kleiner 3,5:6,5 und insbesondere kleiner 3:7 eingesetzt werden. Bei der Bildung all dieser Verhältnisse ist die "Überschussmenge" s₁ an C₅-Alkohol nicht berücksichtigt.

Die Menge s₁ an C₅-Alkohol, welche über die Menge m₁ der in Terephthalsäure oder deren Derivaten einzuführenden C₅-Alkoholfunktionen hinausgeht, kann im Herstellungsverfahren III als Lösungsmittel wirken. Wird in dem Verfahren Wasser gebildet, so wirkt die Menge s₁ an C₅-Alkohol vorzugsweise als Schleppmittel für das Wasser, welches mit C₅-Alkohol als azeotropes Gemisch abdestilliert werden kann. Die Menge s₁ ist mit Vorzug kleiner als m₁ + m₂, mit besonderem Vorzug kleiner als 0,6 · (m₁ + m₂), bevorzugt keiner als 0,5 · (m₁ + m₂), besonders bevorzugt kleiner als 0,4 · (m₁ + m₂), weiter bevorzugt kleiner als 0,3 · (m₁ + m₂) und insbesondere kleiner als 0,25 · (m₁ + m₂). Ist die Menge s₁ größer als 0,05 · (m₁ + m₂), bevorzugt größer als 0,10 · (m₁ + m₂), besonders bevorzugt größer als 0,15 · (m₁ + m₂) und insbesondere größer als 0,20 · (m₁ + m₂), stellt dies eine bevorzugte Ausführungsform des Herstellungsverfahrens III dar. Bevorzugt ist die Menge s₁ kleiner als (m₁ + m₂), mit Vorzug kleiner als 0,6 · (m₁ + m₂), besonders bevorzugt keiner als 0,5 · (m₁ + m₂) und insbesondere kleiner als 0,4 · (m₁ + m₂) und gleichzeitig bevorzugt größer als 0,05 · (m₁ + m₂), besonders bevorzugt größer als 0,10· (m₁ + m₂) und insbesondere größer als 0,15 · (m₁ + m₂).

Ein bevorzugter Gegenstand der vorliegenden Erfindung ist ein Verfahren zur Herstellung eines erfindungsgemäßen Gemisches umfassend Dipentylterephthalat, Di*iso*nonylterephthalat und Pentyl(*iso*nonyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ *lso*nonanol, wobei das Reaktionsgemisch zum Sieden erhitzt wird und
- m₁ und m₂: den Mol-Äquivalenten der in Terephthalsäure oder ihren Derivaten einzuführenden Alkoholresten von Pentanol und *Iso*nonanol entspricht und
- s₁: größer als 0 ist und insbesondere im Bereich des 0,05- bis 0,60-fachen von (m₁ + m₂) liegt.

Bevorzugt weisen dabei die Pentyl-Reste und die *Iso*nonyl-Reste die weiter oben für die erfindungsgemäßen Gemische erläuterten Isomerenzusammensetzungen auf.

In dem erfinderischen Verfahren wird vorzugsweise keine Überschussmenge an Cg-Alkohol eingesetzt, sondern nur die Menge an C₉-Alkohol, welche der Menge der in der Terephthalsäure oder deren Derivaten einzubauenden C₉₋Alkoholresten, insbesondere der Menge der in der Terephthalsäure oder deren Derivaten als Teil von Esterfunktionen einzubauenden C₉-Alkoholresten entspricht. "Keine Überschussmenge" bedeutet hier, dass vorzugsweise weniger als 0,2 Mol-Äquivalente, bevorzugt weniger als 0,1 Mol-Äquivalente und insbesondere weniger als 0,05 Mol-Äquivalente basierend auf der Anzahl der eingesetzten Menge an C₉-Alkohol nicht in eine Esterfunktion eingebaut wird. Dementsprechend werden vorzugsweise mindestens 0,8 Mol-Äquivalente, bevorzugt mindestens 0,9 Mol-Äquivalente und insbesondere mindestens 0,95 Mol-Äquivalente des in dem erfindungsgemäßen Verfahren eingesetzten C₉-Alkohols als C₉-Alkoholrest in Terephthalsäure oder deren Derivat eingeführt.

Die gewünschte Zusammensetzung des herzustellenden Estergemisches kann besonders gut gesteuert und das Verfahrensprodukt nach einem geringeren Aufarbeitungsaufwand direkt als Weichmacher oder Weichmacherkomponente genutzt werden, wenn die in dem erfindungsgemäßen Verfahren eingesetzten Komponenten umfassend Terephthalsäure oder Derivate davon und das C₅- und C₉-Alkoholgemisch weniger als 50 Vol.-%, vorzugsweise weniger als 35 Vol.-% und insbesondere weniger als 20 Vol.-%, weiter bevorzugt weniger als 10 Vol.-% an Komponenten enthält, welche keine Edukte, End- oder Zwischenprodukte der Umsetzung von Terephthalsäure oder Derivaten davon mit den C₅- und C₉-Alkoholen sind. Unter diese Mengenangaben fällt nicht die Menge s₁ an C₅-Alkohol. Unter diese Menge fällt jedoch jeder Alkohol, welcher kein C₅- oder C₉-Alkohol ist. Zur Verbesserung der Steuerbarkeit der Zusammensetzung des aus der Umsetzung des Herstellungsverfahrens II resultierenden Estergemisches enthält die Reaktionsmischung während der Umsetzung mit Vorzug weniger als 0,5 Mol-Äquivalent, bevorzugt weniger als 0,1 Mol-Äquivalent, besonders bevorzugt weniger als 0,05 Mol-Äquivalent und insbesondere weniger als 0,01 Mol-Äquivalent an Alkoholen, welche keine C₅- und keine C₉-Alkohole sind, wobei sich die Mol-Äquivalente auf die Gesamtheit aller in der Reaktionsmischung enthaltenen Alkohole (entsprechend 1 Mol-Äquivalent) beziehen.

In bevorzugten Ausführungsformen ermöglicht das Herstellungsverfahren III die Bereitstellung von erfindungsgemäßen Gemischen, in welchem das Molverhältnis der Ester I, II und III um weniger als 15 Punkte, vorzugsweise um weniger als 10 Punkte vom statistisch ermittelten Erwartungswert abweicht, welcher sich ergibt, wenn man von einem vollständigen Einbau der C₉-Alkoholreste ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Das Herstellungsverfahren III kann in dem Fachmann bekannten, typischen Veresterungsapparaturen unter üblichen Verfahrensbedingungen durchgeführt werden. Vorzugsweise findet das Verfahren bei Temperaturen bei oder oberhalb des Siedepunktes des C₅-Alkohols, des Siedepunktes von Wasser und/oder des Siedepunktes eines aus C₅-Alkohol und Wasser gebildeten Azeotropes statt, so dass die Überschussmenge s₁ des C₅-Alkohols bei dem vorgegebenen Druck aus dem Reaktionsgemisch abdestilliert werden kann. Eine weitere Verbesserung der Steuerbarkeit der Zusammensetzung des aus dem Herstellungsverfahren III resultierenden Estergemisches wird erreicht, wenn das Verfahren in einer Apparatur mit Kolonne durchgeführt wird. Diese gewährleistet vorzugsweise, dass während der Reaktion in der Gasphase befindlicher C₉-Alkohol möglichst quantitativ in das Reaktionsgefäß zurückgeführt wird. Unter dem Begriff "quantitativ" wird in diesem Zusammenhang zu mehr als 80 Mol-%, vorzugsweise zu mehr als 90 Mol-% und insbesondere zu mehr als 95 Mol-% verstanden.

Bevorzugt wird das bei der Reaktion gebildete Wasser aus dem Reaktionsraum entfernt. Vorzugsweise dient dabei der C₅-Alkohol als Schleppmittel. Optional kann in dem erfindungsgemäßen Verfahren weiteres Schleppmittel, beispielsweise Cyclohexan, Toluol, Benzol oder Xylol eingesetzt werden.

Eine Verkürzung der für die Durchführung des erfindungsgemäßen Verfahrens benötigte Zeit wird möglich, wenn die beiden Alkohole R₁OH und R₂OH nicht zeitgleich eingesetzt werden, sondern zumindest Teile des Alkohols R₁OH später als der Alkohol R₂OH zur Terephthalsäure oder deren Derivaten hinzugesetzt wird. Bevorzugt werden deshalb zumindest Teile des Alkohols R₁OH später als der Alkohol R₂OH mit Terephthalsäure oder deren Derivaten zur Reaktion gebracht. Mit Vorzug wird Terephthalsäure (oder deren Derivate) mit dem Alkohol R₂OH und optional einem Katalysator sowie Teilen von R₁OH zum Sieden erhitzt und die verbleibenden Teile des Alkohols R₁OH werden erst zu einem späteren Zeitpunkt zu diesem Reaktionsgemisch gegeben.

Vorzugsweise werden während der Reaktion in regelmäßigen Abständen GC-Chromatogramme angefertigt oder die Säurezahl bestimmt, um den Fortschritt der Reaktion zu beobachten. Mit Vorzug wird die Reaktion durch Abkühlen und/oder Zerstörung des Katalysators, beispielsweise durch Zugabe von Wasser und/oder Base, abgebrochen, nachdem in den GC-Chromatogrammen der Restgehalt an eingesetzter Terephthalsäure oder an dem jeweiligen eingesetzten Säurederivat einen bestimmten Wert unterschreitet. Wird in dem erfindungsgemäßen Verfahren Terephthalsäure eingesetzt, so wird die Reaktion bevorzugt abgebrochen, nachdem die Säurezahl des Reaktionsgemisches einen Wert von 1,00 mg KOH, insbesondere einen Wert von 0,50 mg KOH pro g Reaktionsgemisch unterschreitet. Die Säurezahl kann nach DIN EN ISO 2114 bestimmt werden. Wird in dem erfindungsgemäßen Verfahren ein Terephthalsäurederivat, beispielsweise ein Terephthalsäuredimethylester, eingesetzt, so kann als Grenzpunkt einer im Wesentlichen vollständig abgelaufenen Reaktion, der Gehalt der eingesetzten Komponente selbst oder aber der Gehalt eines Zwischenproduktes, beispielsweise im Fall eines eingesetzten Dimethylesters der Gehalt aller Monomethylester innerhalb des Reaktionsgemisches, vorzugsweise mittels GC, bestimmt werden. Mit Vorzug wird die Reaktion durch Abkühlen und/oder Zerstörung des Katalysators abgebrochen, nachdem in den GC-Chromatogrammen ein Restgehalt an eingesetzter Terephthalsäure oder an eingesetztem Terephthalsäurederivat oder an Zwischenprodukt, wie beispielsweise Monomethylester von kleiner als 5,0 Flächen-%, bevorzugt von kleiner als 2,0 Flächen-% und insbesondere von kleiner 1,0 Flächen-%, bezogen auf die Gesamtfläche aller Ester im GC-Chromatogramm, festgestellt wird.

In einer besonders bevorzugten Ausführungsform des Herstellungsverfahrens III wird ein Katalysator eingesetzt und dieser zerstört, nachdem im Reaktionsgemisch der Gehalt an C₅-Alkohol auf weniger als 15 Vol.-%, vorzugsweise auf weniger als 10 Vol.-% und insbesondere auf weniger als 5 Vol.-% - bezogen auf das Volumen des gesamten Reaktionsgemisches abgesenkt wurde. Mit besonderem Vorzug wird der Gehalt an C₅-Alkohol dabei auf weniger als 3 Vol.-% und insbesondere auf weniger als 1 Vol.-%, bezogen auf das Volumen des gesamten Reaktionsgemisches, vorzugsweise mittels Abdestillierens, abgesenkt. Eine besonders bevorzugte Ausführungsform des erfindungsgemäßen Verfahrens liegt vor, wenn in dem Verfahren ein Katalysator eingesetzt wird und im Reaktionsgemisch der Gehalt an C₅-Alkohol auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des C₅-Alkohol - abgesenkt wurde, bevor der Katalysator zerstört wird. Hierbei findet die Zerstörung des Katalysators vorzugsweise statt, nachdem mittels Reaktionskontrolle ein Reaktionsfortschritt von mindestens 90 % festgestellt wurde, beispielsweise indem in den Reaktionsfortschritt kontrollierenden GC-Chromatogrammen ein Restgehalt an eingesetzter Terephthalsäure oder an eingesetztem Derivat der Säure oder an Zwischenprodukt, wie beispielsweise Monomethylester, von kleiner als 5,0 Flächen-%, insbesondere von kleiner 1,0 Flächen-%, bezogen auf die Gesamtfläche aller Ester im GC-Chromatogramm, festgestellt wird oder indem eine bestimmte Säurezahl unterschritten wird. Durch das Absenken des Gehaltes an C₅-Alkohol wird eine verbesserte Steuerbarkeit des erfindungsgemäßen Verfahrens zu Estergemischen erreicht, deren Zusammensetzungen besonders geringe Abweichungen vom statistischen Erwartungswert aufweisen.

Bevorzugter Gegenstand der vorliegenden Erfindung ist ein Verfahren zur Herstellung eines erfindungsgemäßen Estergemisches durch Umsetzung von Terephthalsäure oder Derivaten davon, welche keine Estergruppen COOR mit einem Rest R enthalten, dessen Alkohol ROH einen höheren Siedepunkt bei einem bestimmten Druck als der Alkohol R₁OH des Restes R₁ bei demselben Druck, mit einer Menge (m₁+ s₁) R₁OH und einer Menge m₂ R₂OH, in welchem
- das Reaktionsgemisch zum Sieden erhitzt wird,
- vorzugsweise die Reaktion abgebrochen wird, nachdem ein Reaktionsfortschritt von mindestens 90 % festgestellt wurde, beispielsweise indem die Säurezahl des Reaktionsgemisches einen Wert von 1,00 mg KOH pro g Reaktionsgemisch, insbesondere einen Wert von 0,50 mg KOH pro g Reaktionsgemisch unterschreitet oder in den GC-Chromatogrammen ein Restgehalt an einer während der Reaktion eingesetzten Komponente oder einem gebildeten und während der Reaktion sich verbrauchenden Zwischenprodukt von kleiner als 5,0 Flächen-%, insbesondere von kleiner 1,0 Flächen-%, bezogen auf die Gesamtfläche aller Ester im GC-Chromatogramm, festgestellt wird,
- vorzugsweise mindestens 0,8 Mol-Äquivalente, bevorzugt mindestens 0,9 Mol-Äquivalente und insbesondere mindestens 0,95 Mol-Äquivalente des in dem erfindungsgemäßen Verfahren eingesetzten Alkohols R₂OH als OR₂-Rest in die Terephthalsäure oder deren Derivat eingeführt wird,
- ein Katalysator eingesetzt und im Reaktionsgemisch der Gehalt an R₁OH auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkohols R₁OH - abgesenkt wurde, bevor der Katalysator zerstört wird,
- und wobei
   - m₁ und m₂: den Mol-Äquivalenten der in der Terephthalsäure oder deren Derivaten einzuführenden OR₁- und OR₂- Alkoholresten entspricht und
   - s₁: größer als 0 ist und insbesondere im Bereich des 0,01- bis 0,60-fachen von (m₁ + m₂) liegt.

Hierbei steht R₂ vorzugsweise für *Iso*nonyl und mit Vorzug weicht zudem das Molverhältnis der Ester I, II und III um weniger als 15 Punkte, vorzugsweise um weniger als 10 Punkte von dem statistisch ermittelten Erwartungswert ab, welcher sich ergibt, wenn man von einem vollständigen Einbau der *Iso*nonyl-Alkoholreste ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Gegenstand der vorliegenden Erfindung ist zudem die Verwendung der Herstellungsverfahren II und III zur Einstellung verarbeitungs- und/oder anwendungsrelevanter Eigenschaften eines Estergemisches durch Steuerung der Mengenverteilung der Ester im Estergemisch. Beispiele für die Beeinflussung einiger im Bereich der Weichmacher verarbeitungs- und anwendungsrelevanter Eigenschaften durch die Wahl der eingesetzten Mengen an C₉-Alkohol im Herstellungsverfahren II beziehungsweise durch die Wahl des m₁:m₂-Verhältnisses im Herstellungsverfahren III sind oben im Text aufgeführt.

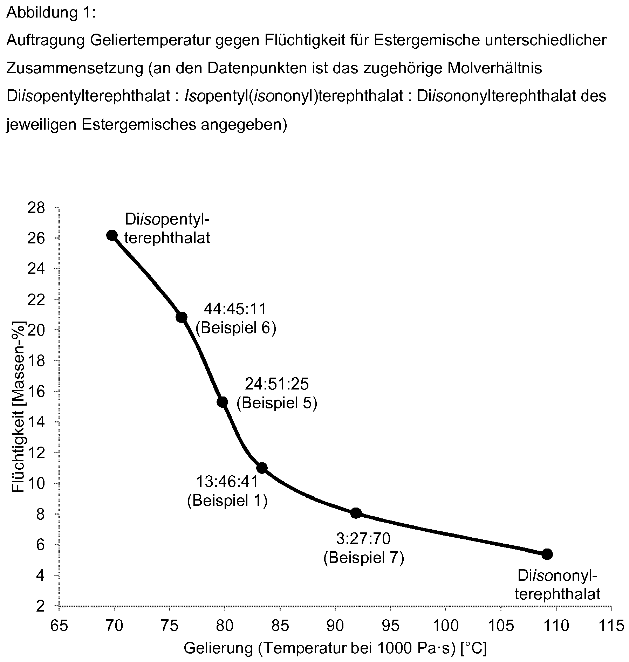

Besonders bevorzugter Gegenstand der vorliegenden Erfindung ist die Verwendung der Herstellungsverfahren II und III zur Steuerung der Geliertemperatur eines Terephthalsäureestergemisch-haltigen Plastisols und/oder der Steuerung der Flüchtigkeit eines Terephthalsäureestergemisch-haltigen Prüfkörpers durch Steuerung der Mengenverteilung der Ester im Estergemisch. In der Abbildung 1 ist für erfindungsgemäße Estergemische mit unterschiedlicher Mengenverteilung der enthaltenen Ester die Geliertemperatur der Plastisole gegen die zugehörige Flüchtigkeit der dasselbe Estergemisch enthaltenden Folien aufgetragen. Aus dieser Abbildung wird ersichtlich, dass Produkte (Plastisole bzw. Folien) unterschiedlicher erfindungsgemäßer Gemische voneinander abweichende Geliertemperaturen und Flüchtigkeiten aufweisen und der Fachmann je nach der für die Verarbeitung und/oder Anwendung benötigten Geliertemperatur und Flüchtigkeit die geeignete Zusammensetzung eines Estergemisches auswählen kann. Selbiges gilt für andere Eigenschaften wie beispielsweise die unter anderem mittels des im experimentellen Teil beschriebenen Testes bestimmbare Verträglichkeit der Estergemische mit Polymeren, die die Weichmacher-Effizienz beschreibenden Shore-Härte, die Veränderung der Viskosität eines entsprechenden Plastisols nach Lagerung oder die Massenveränderung eines entsprechenden Plastisols bei Wasserlagerung. Das erfindungsgemäße Verfahren ermöglicht dem Fachmann nun, gezielt Estergemische mit den gewünschten Eigenschaften herzustellen, da mittels des erfindungsgemäßen Verfahrens gezielt die Mengenverteilung der Ester im Estergemisch eingestellt werden kann.

### Experimenteller Teil:

### Siedebereich der Alkohole:

Die in den Beispielen eingesetzten beziehungsweise zur Synthese der in den Beispielen eingesetzten Ester verwendeten Alkohole wiesen folgende Siedebereiche auf: *Iso*nonanol (Evonik Industries AG, Reinheit > 99 %): 205 bis 215 °C bei 1013 hPa; *Iso*pentanol (Gemisch *n*-Pentanol (Sigma Aldrich, Reinheit > 99 %) und 2-Methylbutanol (Sigma Aldrich, Reinheit > 99 %) im Molverhältnis 1:1): 129 bis 138 °C bei 1013 hPa

### Säurezahl:

Die Säurezahl wurde in Anlehnung an DIN EN ISO 2114 bestimmt.

### GC-Analysen:

Die GC- Analyse erfolgte mit folgenden Parametern:
Kapillarsäule: 30 m DB5; 0,25 mm ID; 0,25 µm Film
Trägergas: Helium
Säulenvordruck: 80 kPa
Split: ca. 23,8 ml/min
Ofentemperaturprogramm (Dauer: 51 min): 50 °C (für 1 min), Aufheizen mit 7,5 °C/min auf 350 °C (Temperatur halten für 1 min)
Injektor: 350 °C
Detektor (FID): 400 °C
Injektionsvolumen: 1,0 µl

Die Identifizierung der Komponenten im Chromatogramm der Probe erfolgte mittels einer Vergleichslösung der relevanten Ester. Im Anschluss erfolgte eine Normierung der Signale im Chromatogramm der Probe auf 100 Flächen-%. Die Stoffmengenverhältnisse wurden in hinreichender Näherung aus den Flächenverhältnissen der einzelnen Signale bestimmt.
Die Reinheit wurde über den Anteil der Produktsignale an den Gesamtflächen im Chromatogramm bestimmt.

### Beispiel 1 (erfindungsgemäß):

### Umesterung von Dimethylterephthalat mit Isopentanol und Isononanol gemäß Herstellungsverfahren III (13 : 46 : 41)

In eine Umesterungsapparatur umfassend Rührkolben mit Rührer, Tauchrohr, Thermometer und 20 cm Raschigringkolonne mit aufgesetztem Destillationskopf wurden Dimethylterephthalat (Sigma Aldrich, Reinheit > 99 %) (m_{b}) und die Alkohole R₁OH (m₁ + s₁) und R₂OH (m₂) eingefüllt. Die Apparatur wurde mindestens eine Stunde mit Stickstoff (6 l/h) über das Tauchrohr gespült. Anschließend wurden 0,25 Gew.-% Tetra-n-Butyltitanat (Sigma Aldrich, Reinheit > 97 %) bezogen auf die Masse des Terephthalsäureesters hinzugegeben. Das Gemisch wurde anschließend zum Sieden erhitzt und leichtsiedende Komponenten abdestilliert. Bei sprunghaftem Anstieg der Kopftemperatur wurde die Destillation durch Verschluss des Ablaufhahns unterbrochen und die Reaktion bis zur Einstellung einer stabilen Siedetemperatur am Rückfluss belassen. Während der Reaktion stieg die Sumpftemperatur von T¹ auf T² an. Während der Reaktion wurden stündlich GC-Chromatogramme angefertigt. Sobald in diesen ein Restgehalt an Monomethylester von kleiner als 0,5 Flächen-% bezogen auf die Gesamtfläche aller Ester im GC-Chromatogramm festgestellt wurde, wurden die verbliebenen leichtflüchtigen Komponenten des Reaktionsgemisches unter Vakuum (ca. 1 mbar) bei einer Sumpftemperatur von T³ abdestilliert, so dass der Restgehalt am Alkohol R₁OH unterhalb von 5 Mol-% - bezogen auf die Überschussmenge s₁ des Alkohols R₁OH (gemäß GC) - betrug. Anschließend wurde der Kolbeninhalt bei ausgeschalteter Heizung durch Einleiten von Stickstoff bei 20 mbar bis auf ca. 80 °C abgekühlt. Vom Kolbeninhalt wurde die Säurezahl bestimmt. Entsprechend des Ergebnisses wurde das Reaktionsmedium durch langsames Zutropfen der dreifachen stöchiometrischen Menge an Base (10 %-ige wässrige NaOH-Lösung) neutralisiert und unter Einleitung von Stickstoff (6 l/h) bei 80 °C für 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurde. Der Stickstoffstrom wird so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Produkt über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde eine GC-Analyse durchgeführt, an Hand derer die Reinheit (R) und die Zusammensetzung des Produkts analysiert wurde.

**Tabelle 3: gemessene und berechnete Werte zum Beispiel 1 (Herstellungsverfahren III)**

| | |
|---|---|
| R₁OH = *Iso*pentanol | m₁ + s₁ = 441 g |
| Stoffmenge R₁OH = Stoffmenge R₁-Äquivalente (C₅) | 5 mol |
| R₂OH = *Iso*nonanol | m₂ = 720 g |
| Stoffmenge R₂OH = Stoffmenge R₂-Äquivalente (C₉) | 5 mol |
| Dimethylterephthalat | m_{b} = 776 g |
| Stoffmenge | 4 mol |
| → Stoffmenge Esterfunktionen | 8 mol |
| Erwarteter Molanteil der R₁-Funktionen an R₁ und R₂-Funktionen im Estergemisch (bei vollständigem Einbau von R₂) | 8 mol - 5 mol = 3 mol entsprechend 37,5 Mol-% |
| Verhältnis m₁ : m₂ | 3 : 5 entsprechend 0,375 : 0,625 |
| s₁ | 5 mol - (8 mol - 5 mol) = 2 mol |
| T¹ → T² (Sumpftemperatur Anfang → Ende) | 139 °C → 215 °C |
| T³ | 190 °C |
| R (Reinheit) | 99,7 % |
| Statistische Erwartungswerte für vollständigen Einbau von C₉: (C₅/C₅) : (C₅/C₉) : (C₉/C₉) | **14,1 : 46,8 : 39,1** (erwarteter C₅-Anteil: 37,5 %) |
| Zusammensetzung gemäß GC | **13,4 : 45,8 : 40,8** (C₅-Anteil: 36 %) |
| (C₅/C₅) : (C₅/C₉) : (C₉/C₉) | Abweichung: \|14,1-13,4\| + \|46,8-45,8\| + \|39,1-40,8\| = 3,4 |

Es werden 1270 g aufgearbeitetes Estergemisch **13:46:41** (C₅/C₉ TM 13:46:41) erhalten.

### Beispiel 2 (erfindungsgemäß):

### Umesterung von Dimethylterephthalat mit Isopentanol und Isononanol gemäß Herstellungsverfahren III (1 : 19 : 81)

Das Beispiel 2 wird ausgeführt wie für das Beispiel 1 beschrieben.

**Tabelle 4: gemessene und berechnete Werte zum Beispiel 2 (Herstellungsverfahren III)**

| | |
|---|---|
| R₁OH = *Iso*pentanol | m₁ + s₁ = 308,5 g |
| Stoffmenge R₁OH = Stoffmenge R₁-Äquivalente (C₅) | 3,5 mol |
| R₂OH = *Iso*nonanol | m₂ = 1296 g |
| Stoffmenge R₂OH = Stoffmenge R₂-Äquivalente (C₉) | 9 mol |
| Dimethylterephthalat | m_{b} = 970 g |
| Stoffmenge | 5 mol |
| → Stoffmenge Esterfunktionen | 10 mol |
| Erwarteter Molanteil R₁-Funktionen an R₁ und R₂-Funktionen im Estergemisch (bei vollständigem Einbau von R₂) | 10 mol - 9 mol = 1 mol entsprechend 10 Mol-% |
| Verhältnis m₁ : m₂ | 0,1 : 0,9 |
| s₁ | 3,5 mol - (10 mol - 9 mol) = 2,5 mol |
| T¹ → T² (Sumpftemperatur Anfang → Ende) | 131 °C → 200 °C |
| T³ | 200 °C |
| R (Reinheit) | > 99,9 % |
| Statistische Erwartungswerte für vollständigen Einbau von C₉: (C₅/C₅) : (C₅/C₉) : (C₉/C₉) | **1,0 : 18,0 : 81,0** (erwarteter C₅-Anteil: 10 %) |
| Zusammensetzung gemäß GC | **1,0 : 18,5 : 80,5** (C₅-Anteil: 10,3 %) |
| (C₅/C₅) : (C₅/C₉) : (C₉/C₉) | Abweichung: \|1-1\| + \|18,5-18,0\| + \|80,5-81,0\| = 1 |

Es werden 1902 g aufgearbeitetes Estergemisch 1:19:81 erhalten.

### Beispiel 3 (erfindungsgemäß):

### Umesterung von Diisopentylterephthalat mit Isononanol gemäß Herstellungsverfahren II (11 : 45 : 44)

Das Beispiel 3 wurde ausgeführt wie für das Beispiel 1 beschrieben, wobei jedoch statt *Iso*pentanol, *Iso*nonanol und Dimethylterephthalat als Edukte Di*iso*pentylterephthalat (Ester Iin einer Menge m_{E}) und *Iso*nonanol (in der Menge m_{R2}) mit dem angegebenen Katalysator (in angegebener Katalysatormenge) eingesetzt wurden. Die Reaktion wurde durch Abkühlen abgebrochen, sobald in den stündlich aufgenommenen GC-Chromatogrammen ein Restgehalt an *Iso*nonanol von kleiner als 0,5 Flächen-% bezogen auf die Gesamtfläche aller Terephthalsäureester festgestellt wurde und die verbliebenen leichtflüchtigen Komponenten unter Vakuum (ca. 1 mbar) soweit abdestilliert wurden, dass der Restgehalt an *Iso*pentanol unterhalb von 5 Mol-%, bezogen auf die Gesamtmenge des bei der Reaktion gebildeten *Iso*pentanols lag (berechnet über Flächen-% im GC bezogen auf *Iso*nonyl-Alkoholrest-haltige Terephthalsäureester).

**Tabelle 5: gemessene und berechnete Werte zum Beispiel 3 (Herstellungsverfahren II)**

| | |
|---|---|
| m_{E} (Di*iso*pentylterephthalat) | 1697 g (5,5 mol) |
| Stoffmenge R₁-Äquivalente (C₅) | 11 mol |
| m_{R2} (*Iso*nonanol) | 1025 g |
| Stoffmenge R₂OH = Stoffmenge R₂-Äquivalente (C₉) | 7,1 mol |
| Eingesetztes Mengenverhältnis R₁-Äquivalente zu R₂-Äquivalente (C₅ : C₉) | 11,0 : 7,1 entsprechend 2,0 : 1,3 |
| T¹ → T² (Sumpftemperatur Anfang → Ende) | 170 °C → 202 °C |
| R (Reinheit) | > 99,9 % |
| Statistische Erwartungswerte für vollständigen Einbau von C₉: (C₅/C₅) : (C₅/C₉) : (C₉/C₉) | **12 : 46 : 42** (erwarteter C₅-Anteil: 35 %) |
| Zusammensetzung gemäß GC (C₅/C₅) : (C₅/C₉) : (C₉/C₉) | **11 : 45 : 44** (C₅-Anteil: 34 %) Abweichung: \|12-11\| + \|46-45\| + \|42-44\| = 4 |

Es werden 2102 g aufgearbeitetes Estergemisch 11:45:44 erhalten.

### Beispiel 4 (erfindungsgemäß):

### Umesterung von Dimethylterephthalat mit Isopentanol und Isononanol gemäß Herstellungsverfahren III (97 : 3 : 0)

Das Beispiel 4 wird ausgeführt wie für das Beispiel 1 beschrieben.

**Tabelle 6: gemessene und berechnete Werte zum Beispiel 4 (Herstellungsverfahren III)**

| | |
|---|---|
| R₁OH = *Iso*pentanol | m₁ + s₁ = 436 g |
| Stoffmenge R₁OH = Stoffmenge R₁-Äquivalente (C₅) | 4,95 mol |
| R₂OH = *Iso*nonanol | m₂ = 7,2 g |
| Stoffmenge R₂OH = Stoffmenge R₂-Äquivalente (C₉) | 0,05 mol |
| Dimethylterephthalat | m_{b} = 388 g |
| Stoffmenge | 2 mol |
| → Stoffmenge Esterfunktionen | 4 mol |
| Erwarteter Molanteil R₁-Funktionen an R₁ und R₂-Funktionen im Estergemisch (bei vollständigem Einbau von R₂) | 4 mol - 0,05 mol = 3,95 mol entsprechend 10 Mol-% |
| Verhältnis m₁ : m₂ | 3,95 : 0,05 entsprechend 0,988 : 0,012 |
| s₁ | 4,95 mol - (4 mol - 0,05 mol) = 1 mol |
| T¹ → T² (Sumpftemperatur Anfang → Ende) | 124 °C → 195 °C |
| T³ | 190 °C |
| R (Reinheit) | 99,7 % |
| Statistische Erwartungswerte für vollständigen Einbau von C₉: (C₅/C₅) : (C₅/C₉) : (C₉/C₉) | **97,6 : 2,4 : <0,1** (erwarteter C₅-Anteil: 98,7 %) |
| Zusammensetzung gemäß GC (C₅/C₅) : (C₅/C₉) : (C₉/C₉) | **97 : 3 : 0** (C₅-Anteil: 98,5 %) Abweichung: \|97,6-97\| + \|2,4-3,0\| = 1,2 |

Es werden 652 g aufgearbeitetes Estergemisch 97:3:0 erhalten.

### Beispiel 5 (erfindungsgemäß):

### Umesterung von Dimethylterephthalat mit Isopentanol und Isononanol gemäß Herstellungsverfahren III (24 : 51 : 25)

Das Beispiel 5 wird ausgeführt wie für das Beispiel 1 beschrieben.

**Tabelle 7: gemessene und berechnete Werte zum Beispiel 5 (Herstellungsverfahren III)**

| | |
|---|---|
| R₁OH = *Iso*pentanol | m₁ + s₁ = 705 g |
| Stoffmenge R₁OH = Stoffmenge R₁-Äquivalente (C₅) | 8 mol |
| R₂OH = *Iso*nonanol | m₂ = 576 g |
| Stoffmenge R₂OH = Stoffmenge R₂-Äquivalente (C₉) | 4 mol |
| Dimethylterephthalat | m_{b} = 776 g |
| Stoffmenge | 4 mol |
| → Stoffmenge Esterfunktionen | 8 mol |
| Erwarteter Molanteil R₁-Funktionen an R₁ und R₂-Funktionen im Estergemisch (bei vollständigem Einbau von R₂) | 8 mol - 4 mol = 4 mol entsprechend 50 Mol-% |
| Verhältnis m₁ : m₂ | 0,5 : 0,5 |
| s₁ | 8 mol - (8 mol - 4 mol) = 4 mol |
| T¹ → T² (Sumpftemperatur Anfang → Ende) | 123 °C → 174 °C |
| T³ | 180 °C |
| R (Reinheit) | 99,9 % |
| Statistische Erwartungswerte für vollständigen Einbau von C₉: (C₅/C₅) : (C₅/C₉) : (C₉/C₉) | **25 : 50 : 25** (erwarteter C₅-Anteil: 50 %) |
| Zusammensetzung gemäß GC (C₅/C₅) : (C₅/C₉) : (C₉/C₉) | **24 : 51 : 25** (C₅-Anteil: 49,5 %) Abweichung: \|25-24\| + \|51-50\| + \|25-25\| = 2 |

Es werden 1254 g aufgearbeitetes Estergemisch **24:51:25** (C₅/C₉ TM 24:51:25) erhalten.

### Beispiel 6 (erfindungsgemäß):

### Umesterung von Dimethylterephthalat mit Isopentanol und Isononanol gemäß Herstellungsverfahren III (44 : 45 : 11)

Das Beispiel 6 wird ausgeführt wie für das Beispiel 1 beschrieben.

**Tabelle 8: gemessene und berechnete Werte zum Beispiel 6 (Herstellungsverfahren III)**

| | |
|---|---|
| R₁OH = *Iso*pentanol | m₁ + s₁ = 846 g |
| Stoffmenge R₁OH = Stoffmenge R₁-Äquivalente (C₅) | 9,6 mol |
| R₂OH = *Iso*nonanol | m₂ = 346 g |
| Stoffmenge R₂OH = Stoffmenge R₂-Äquivalente (C₉) | 2,4 mol |
| Dimethylterephthalat | m_{b} = 776 g |
| Stoffmenge | 4 mol |
| → Stoffmenge Esterfunktionen | 8 mol |
| Erwarteter Molanteil R₁-Funktionen an R₁ und R₂-Funktionen im Estergemisch (bei vollständigem Einbau von R₂) | 8 mol - 2,4 mol = 5,6 mol entsprechend 70 Mol-% |
| Verhältnis m₁ : m₂ | 5,6 : 2,4 entsprechend 0,7 : 0,3 |
| s₁ | 9,6 mol - (8 mol - 2,4 mol) = 4 mol |
| T¹ → T² (Sumpftemperatur Anfang → Ende) | 123 °C → 166 °C |
| T³ | 190 °C |
| R (Reinheit) | 99,8 % |
| Statistische Erwartungswerte für vollständigen Einbau von C₉: (C₅/C₅) : (C₅/C₉) : (C₉/C₉) | **49 : 42 : 9** (erwarteter C₅-Anteil: 70 %) |
| Zusammensetzung gemäß GC | **44 : 45 : 11** (C₅-Anteil: 66,5 %) |
| (C₅/C₅) : (C₅/C₉) : (C₉/C₉) | Abweichung: \|49-44\| + \|45-42\| + \|11-9\| = 10 |

Es werden 1174 g aufgearbeitetes Estergemisch **44:45:11** (C₅/C₉ TM 44:45:11) erhalten.

### Beispiel 7 (erfindungsgemäß):

### Umesterung von Dimethylterephthalat mit Pentanol und Isononanol (3:27:70)

Das Beispiel 7 wird ausgeführt wie für das Beispiel 1 beschrieben. Es wird ein Estergemisch mit einer Zusammensetzung (C₅/C₅) : (C₅/C₉) : (C₉/C₉) von **3:27:70** (C₅/C₉ TM 3:27:70) gemäß GC erhalten.

### Gegenüberstellung verarbeitungs- und anwendungsrelevanter Eigenschaften erfinderischer Gemische und nicht erfindungsgemäßer Gemische

Untersucht wurden folgende Gemische umfassend die Terephthalsäureester der Formeln I, II und III mit der Definition der Reste R₁ und R₂ wie in Tabelle 9 angegeben.

Die Gemische umfassend den Mischester (im Folgenden abgekürzt als "TM") wurden wie in Beispiel 1 erläutert - jedoch mit den entsprechenden Alkoholen - hergestellt. Eingesetzt wurden folgende Alkohole:
Ethylhexanol: 2-Ethylhexanol von Sigma Aldrich, Reinheit > 99%
Butanol: *n*-Butanol von Sigma Aldrich, Reinheit > 99%
Propylheptanol: 2-Propylheptanol von Evonik Industries, Gehalt an C10-Alkoholen > 99% Tridecanol: *Iso*tridecanol Marlipal 013 von Sasol, Reinheit > 99% C13-Alkohole

Die Mischester-freien Gemische (im Folgenden abgekürzt als "Blend") wurden durch Abmischung der jeweiligen Terephthalate hergestellt. Folgende Terephthalate wurden eingesetzt:
DINT: hergestellt durch Umesterung von Dimethylterephthalat (Sigma Aldrich, Reinheit > 99 %) mit *Iso*nonanol (wie am Anfang des experimentellen Teils angegeben);
DPT: hergestellt durch Umesterung von Dimethylterephthalat (Sigma Aldrich, Reinheit > 99 %) mit *Iso*pentanol (wie am Anfang des experimentellen Teils angegeben);
DEHT: Eastman 168, Eastman, Estergehalt > 99%;
Dibutylterephthalat: Di(*n*-butyl)terephthalat Eastman DBT, Eastman, Estergehalt > 99%.

**Tabelle 9: untersuchte Terephthalsäureestergemische (im Weiteren bezeichnet als "jeweiliger Weichmacher aus Tabelle 9")**

| R₁ | R₂ | Molverhältnis der Ester I : II : III | | Bezeichnung |
|---|---|---|---|---|
| Ethylhexyl | Ethylhexyl | | | DEHT |
| *Iso*nonyl | *Iso*nonyl | | | DINT |
| *Iso*pentyl | *Iso*pentyl | | | DPT |
| | | | | |
| Butyl | Ethylhexyl | 3:27:70 | hergestellt analog Beispiel 1 | C₄/C₈ TM 3:27:70 |
| Butyl | Ethylhexyl | 11:45:24 | II | C₄/C₈ TM 11:45:24 |
| Butyl | Ethylhexyl | 26:50:24 | II | C₄/C₈ TM 26:50:24 |
| | | | | |
| *Iso*pentyl | *Iso*nonyl | 3:27:70 | Beispiel 7 | C₅/C₉ TM 3:27:70* |
| *Iso*pentyl | *Iso*nonyl | 13:46:41 | Beispiel 1 | C₅/C₉ TM 13:46:41* |
| *Iso*pentyl | *Iso*nonyl | 24:51:25 | Beispiel 5 | C₅/C₉ TM 24:51:25* |
| *Iso*pentyl | *Iso*nonyl | 44:45:11 | Beispiel 6 | C₅/C₉ TM 44:45:11* |
| | | | | |
| *Iso*pentyl | Propylheptyl | 53:40:7 | hergestellt analog Beispiel 1 | C₅/C₁₀ TM 53:40:7 |
| *Iso*pentyl | Propylheptyl | 36:49:15 | II | C₅/C₁₀ TM 36:49:15 |
| *Iso*pentyl | Propylheptyl | 18:50:32 | II | C₅/C₁₀ TM 18:50:32 |
| *Iso*pentyl | | | | |
| *Iso*pentyl | Tridecyl | 49:44:7 | II | C₅/C₁₃ TM 49:44:7 |
| *Iso*pentyl | Tridecyl | 29:52:19 | II | C₅/C₁₃ TM 29:52:19 |
| *Iso*pentyl | Tridecyl | 13:51:36 | II | C₅/C₁₃ TM 13:51:36 |
| | | | | |
| Ethylhexyl | *Iso*nonyl | 7:38:55 | II | C₈/C₉ TM 7:38:55 |
| Ethylhexyl | *Iso*nonyl | 20:46:34 | II | C₈/C₉ TM 20:46:34 |
| Ethylhexyl | *Iso*nonyl | 38:48:14 | II | C₈/C₉ TM 38:48:14 |
| | | | | |
| Ethylhexyl | Propylheptyl | 7:38:55 | II | C₈/C₁₀ TM 7:38:55 |
| Ethylhexyl | Propylheptyl | 17:49:34 | II | C₈/C₁₀ TM 17:49:34 |
| Ethylhexyl | Propylheptyl | 35:48:17 | II | C₈/C₁₀ TM 35:48:17 |
| | | | | |
| Butyl | *Iso*nonyl | 10:0:90 | Mischung von DINT und Dibutylterephthalat | C₄/C₉ Blend 10:90 |
| Butyl | *Iso*nonyl | 20:0:80 | II | C₄/C₉ Blend 20:80 |
| Butyl | *Iso*nonyl | 40:0:60 | II | C₄/C₉ Blend 40:60 |
| Butyl | *Iso*nonyl | 60:0:40 | II | C₄/C₉ Blend 60:40 |
| Butyl | *Iso*nonyl | 80:0:20 | II | C₄/C₉ Blend 80:20 |
| | | | | |
| *Iso*pentyl | *Iso*nonyl | 80:0:20 | Mischung von DINT und DPT | C₅/C₉ Blend 80:20 |
| *Iso*pentyl | *Iso*nonyl | 60:0:40 | II | C₅/C₉ Blend 60:40 |
| *Iso*pentyl | *Iso*nonyl | 40:0:60 | II | C₅/C₉ Blend 40:60 |
| *Iso*pentyl | *Iso*nonyl | 20:0:80 | II | C₅/C₉ Blend 20:80 |

| | | | | |
|---|---|---|---|---|
| * erfindungsgemäß | | | | |

### Beispiel 8:

### Herstellung von Plastisolen der Gemische

Es wurden PVC-Plastisole hergestellt, wie sie beispielsweise zur Fertigung von Deckstrichfilmen für Fußbodenbeläge verwendet werden. Die Angaben in den Plastisolrezepturen sind jeweils in Massenanteilen. Die Rezepturen der Polymerzusammensetzungen sind in Tabelle 10 aufgelistet.

**Tabelle 10: Plastisolrezeptur**

| | |
|---|---|
| PVC (Vestolit B 7021 - Ultra; Fa. Vestolit) | 100 |
| jeweiliger Weichmacher aus Tabelle 9 | 50 |
| epoxidiertes Sojabohnenöl als Co-Stabilisator (Drapex 39, Fa. Galata) | 3 |
| Thermostabilisator auf Ca/Zn-Basis (Mark CZ 149, Fa. Galata) | 2 |

| | |
|---|---|
| Angaben in phr (phr = parts per hundred parts resin) | |

Die Estergemische wurden vor der Zugabe auf 25 °C temperiert. Zuerst wurden die flüssigen und dann die pulverförmigen Bestandteile in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sichergestellt, dass die Homogenisierung des Plastisols bei einem definierten Energieeintrag erreicht wurde. Danach wurde das Plastisol sofort für weitere Untersuchungen in einem Klimaschrank auf 25,0 °C temperiert.

### Beispiel 9:

### Geliertemperatur der Plastisole

Die Untersuchung des Gelierverhaltens der Plastisole wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

**Folgende Parameter wurden eingestellt:**

| Modus: | Temperatur-Gradient | |
|---|---|---|
| | Start-Temperatur: | 25 °C |
| | End-Temperatur: | 180 °C |
| | Heiz/Kühlrate: | 5 °C / min |
| | Oszillations-Frequenz: | 4 - 0,1 Hz Rampe logarithmisch |
| | Kreisfrequenz Omega: | 10 1 / s |
| | Anzahl Messpunkte: | 63 |
| | Messpunktdauer: | 0,5 min |
| | Automatische Spaltnachführung F : | 0 N |
| | Konstante Messpunktdauer Spaltweite | 0,5 mm |

### Durchführung der Messung:

Auf die untere Messsystemplatte wurden mit dem Spatel einige Gramm des zu messenden Plastisols luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als 6 mm rundum). Anschließend wurde die Temperierhaube über der Probe positioniert und die Messung gestartet. Bestimmt wurde die sogenannte komplexe Viskosität des Plastisols nach 24 h (Lagerung des Plastisols bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Plastisolviskosität von 1000 Pa·s verwendet.

**Tabelle 11: Gelierung der Plastisole nach 24 h, Temperatur in °C bei Erreichen einer Plastisolviskosität von 10³ Pa·s (kurz: Geliertemperatur)**

| Weichmacher | Geliertemperatur [°C] | Weichmacher | Geliertemperatur [°C] |
|---|---|---|---|
| DEHT | 87,3 | C₈/C₉ TM 7:38:55 | 106,3 |
| DINT | 109,2 | C₈/C₉ TM 20:46:34 | 100,3 |
| DPT | 69,8 | C₈/C₉ TM 38:48:14 | 96,0 |
| | | | |
| C₄/C₈ TM 3:27:70 | 82,4 | C₈/C₁₀ TM 7:38:55 | 126,7 |
| C₄/C₈ TM 11:45:24 | 77,6 | C₈/C₁₀ TM 17:49:34 | 118,9 |
| C₄/C₈ TM 26:50:24 | 72,7 | C₈/C₁₀ TM 35:48:17 | 109,0 |
| | | | |
| C₅/C₉ TM 3:27:70* | 91,9 | C₄/C₉ Blend 10:90 | 100,3 |
| C₅/C₉ TM 13:46:41* | 83,4 | C₄/C₉ Blend 20:80 | 88,0 |
| C₅/C₉ TM 24:51:25* | 79,8 | C₄/C₉ Blend 40:60 | 77,8 |
| C₅/C₉ TM 44:45:11* | 76,1 | C₄/C₉ Blend 60:40 | 71,4 |
| | | C₄/C₉ Blend 80:20 | 66,7 |
| C₅/C₁₀ TM 53:40:7 | 76,1 | | |
| C₅/C₁₀ TM 36:49:15 | 80,0 | C₅/C₉ Blend 80:20 | 73,2 |
| C₅/C₁₀ TM 18:50:32 | 86,4 | C₅/C₉ Blend 60:40 | 76,7 |
| | | C₅/C₉ Blend 40:60 | 81,8 |
| C₅/C₁₃ TM 49:44:7 | 79,7 | C₅/C₉ Blend 20:80 | 89,6 |
| C₅/C₁₃ TM 29:52:19 | 90,0 | | |
| C₅/C₁₃ TM 13:51:36 | 119,1 | | |

| | | | |
|---|---|---|---|
| * erfindungsgemäß | | | |

In der Abbildung 1 ist für die erfindungsgemäßen Estergemische die Geliertemperatur des Plastisols aus der Tabelle 11 gegen die zugehörige Flüchtigkeit der dasselbe Estergemisch enthaltenden Folie (aus Beispiel 11, Tabelle 12) aufgetragen.

### Beispiel 10:

### Herstellung von Folien der Estergemische

Die im Beispiel 8 hergestellten Plastisole wurden jeweils zu 1 mm dicken Folien verarbeitet.

Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 3 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

Aus den Folien wurden 3 Kreise von 10 cm² pro zu prüfender Rezeptur ausgestanzt. Zusätzlich wurden die Kreise mit der Schere radial angeschnitten (2 Schnitte a 5 mm). Die Kreise wurden für eine halbe Stunde im Exsikkator (gefüllt mit KC- Trockenperlen orange) klimatisiert und anschließend ausgewogen.

### Beispiel 11:

### Massenverlust bei Aktivkohlelagerung bei den Folien aus Beispiel 10

Weißblechdosen (1 L, hohe Form) wurden, damit ein Druckaustausch stattfinden konnte, im Deckel mit einem Loch versehen. Der Boden der Weißblechdosen wurde mit 120 mL Aktivkohle bedeckt. Die in diesem Test eingesetzte Aktivkohle (Nr. 774408 der Fa. Roth) wurde vorher in einer Abdampfschale für 6 Stunden im Trockenschrank bei 100 +/-1 °C getrocknet und nach kurzer Abkühlung eingesetzt. Auf die Aktivkohle wurde der erste Probenkreis mittig platziert. Weitere 120 mL Aktivkohle wurden auf den Probenkreis gegeben. Insgesamt wurden die Weißblechdosen mit 480 mL Aktivkohle und 3 Probenkreisen schichtweise befüllt. Der Deckel der Weißblechdosen wurde ohne Druck auf die Dosen aufgelegt.

Die befüllten Weißblechdosen wurden bei 100 +/-1 °C für 3 Tage im Temperierschrank gelagert. Nach der Lagerung wurde die Aktivkohle von den Kreisen mittels Analysenpinsel entfernt, die Kreise zum Abkühlen 30 Minuten in einem Exsikkator gelagert und anschließend ausgewogen.

Nach der Auswaage wurden die Probenkreise wieder mit Aktivkohle zurück in die Weißblechdosen geschichtet. Hierzu wurde darauf geachtet, dass die Probenkreise wieder derselben Aktivkohle und derselben Dose zugeordnet wurden. Die Dosen wurden erneut im Temperierschrank platziert. Nach insgesamt 7 Tagen wurden die Proben dann, wie bereits beschrieben, nochmals ausgewogen.

Es wurde die prozentuale Massenveränderung von jedem Probenkreis berechnet und der Mittelwert über die 3 Kreise je Rezeptur berechnet.

**Tabelle 12: Massenverlust bei Aktivkohlelagerung in Massen-% (Flüchtigkeit)**

| Weichmacher | Flüchtigkeit [Massen-%] | Weichmacher | Flüchtigkeit [Massen-%] |
|---|---|---|---|
| DEHT | 10,4 | C₈/C₉ TM 7:38:55 | 5,3 |
| DINT | 5,4 | C₈/C₉ TM 20:46:34 | 5,9 |
| DPT | 26,2 | C₈/C₉ TM 38:48:14 | 6,9 |
| | | | |
| C₄/C₈ TM 3:27:70 | 16,1 | C₈/C₁₀ TM 7:38:55 | 3,5 |
| C₄/C₈ TM 11:45:24 | 19,5 | C₈/C₁₀ TM 17:49:34 | 4,8 |
| C₄/C₈ TM 26:50:24 | 23,7 | C₈/C₁₀ TM 35:48:17 | 5,4 |
| | | | |
| C₅/C₉ TM 3:27:70* | 8,1 | C₄/C₉ Blend 10:90 | 7,2 |
| C₅/C₉ TM 13:46:41* | 11,0 | C₄/C₉ Blend 20:80 | 10,2 |
| C₅/C₉ TM 24:51:25* | 15,3 | C₄/C₉ Blend 40:60 | 16,2 |
| C₅/C₉ TM 44:45:11* | 20,8 | C₄/C₉ Blend 60:40 | 22,0 |
| | | C₄/C₉ Blend 80:20 | 27,1 |
| C₅/C₁₀ TM 53:40:7 | 22,3 | | |
| C₅/C₁₀ TM 36:49:15 | 20,3 | C₅/C₉ Blend 80:20 | 24,7 |
| C₅/C₁₀ TM 18:50:32 | 15,5 | C₅/C₉ Blend 60:40 | 20,7 |
| | | C₅/C₉ Blend 40:60 | 15,5 |
| C₅/C₁₃ TM 49:44:7 | 18,4 | C₅/C₉ Blend 20:80 | 10,0 |
| C₅/C₁₃ TM 29:52:19 | 11,8 | | |
| C₅/C₁₃ TM 13:51:36 | 7,2 | | |

| | | | |
|---|---|---|---|
| * erfindungsgemäß | | | |

In der Abbildung 1 ist für die erfindungsgemäßen Estergemische die Geliertemperatur des Plastisols aus der Tabelle 11 des Beispiels 9 gegen die zugehörige Flüchtigkeit der dasselbe Estergemisch enthaltenen Folie (aus Tabelle 12) aufgetragen.

Die Abbildung 2 zeigt für alle in der Tabelle 9 enthaltenen Weichmachersysteme eine Auftragung der Geliertemperatur gegen die Flüchtigkeit. Aus dieser Auftragung wird ersichtlich, dass Produkte (Plastisole / Folien) der erfindungsgemäßen Estergemische im Vergleich zu den anderen Polymer-kompatiblen, SVOC-freien Weichmachersystemen niedrigere Geliertemperaturen und gleichzeitig niedrigere Flüchtigkeiten aufweisen und somit gegenüber diesen Weichmachersystemen verbesserte Eigenschaften für Verarbeitung und Anwendung zeigen. Unterhalb Geliertemperaturen von ca. 80 °C weisen ausschließlich Gemische aus Dibutylterephthalat und Di*iso*nonylterephthalat ein besseres Verhältnis von Flüchtigkeit und Geliereigenschaften auf als die erfindungsgemäßen Gemische. Diese C₄-Alkoholrest-haltigen Gemische unterliegen jedoch den in der Beschreibung bereits erläuterten Einschränkungen, da Dibutylterephthalat als SVOC-Komponente einzustufen ist und somit keine erwägbare Alternative oder gar Verbesserung gegenüber den erfindungsgemäßen Gemischen darstellt. Oberhalb Geliertemperaturen von ca. 94 °C scheint gemäß Abbildung 2 ein Estergemisch mit C₈- und C₉-Alkylresten ein besseres Verhältnis von Flüchtigkeit und Geliereigenschaften aufzuweisen als die erfindungsgemäßen Gemische. Wie im Folgenden noch gezeigt werden wird (Beispiel 12), weisen die Estergemische mit C₈- und C₉-Alkylresten jedoch eine schlechte Verträglichkeit mit Polymeren und eine geringere Effizienz (siehe Beispiel 14 zur Shore Härte) auf.

### Beispiel 12:

### Bestimmung der Verträglichkeit der Gemische mit Polymeren gemäß "Loop-Test" (in Anlehnung an ASTM 03291)

Aus den PVC Folien des Beispiels 10 wurden pro Probe jeweils 3 Rechtecke (5 x 1 cm) ausgeschnitten. Die Rechtecke wurden auf der Rückseite beschriftet.

Die Probekörper wurden mit der Hand 180° gebogen und in einer Testschiene platziert. Die Rechtecke aus Folien wurden in Richtung Oberseite gebogen. Zwischen den Probekörpern wird etwas Luft gelassen (ca. 0,5 cm), so dass sich die Proben nicht berühren. Die Enden der gebogenen Rechtecke schauen etwas aus der Schiene heraus.

Die Loop Testschienen wurden im Klimaraum gelagert (Lufttemperatur 22 °C +/-1 °C; Luftfeuchtigkeit 50 % +-5 %).

Nach 1, 7 und 14 Tagen wurden die entsprechenden Rechtecke aus der Schiene genommen und mit der Hand in die entgegengesetzte Richtung gebogen. Die Stelle, an der die Probenkörper am stärksten gebogen waren, wurde jeweils über einem Stück Zigarettenpapier abgestreift und über die Größe des auf dem Zigarettenpapieres entstandenen Fleckes wurde das Ausschwitzverhalten des getesteten Weichmachers wie folgt beurteilt:
- 0: = nicht ausgeschwitzt
- 0,5: = sehr wenig ausgeschwitzt
- 1: = wenig ausgeschwitzt
- 2: = mittel ausgeschwitzt
- 3: = stark ausgeschwitzt
- >3: = sehr stark ausgeschwitzt

**Tabelle 13: Beurteilung des Ausschwitzverhaltens**

| Weichmacher | Beurteilung | | |
|---|---|---|---|
| | nach 1 Tag | nach 7 Tagen | nach 14 Tagen |
| DEHT | 0 | 0 | 0 |
| DINT | 0,5 | 2,5 | 2 |
| DPT | 0 | 0 | 0 |
| | | | |
| C₄/C₈ TM 3:27:70 | 0 | 0 | 0 |
| C₄/C₈ TM 11:45:24 | 0 | 0 | 0 |
| C₄/C₈ TM 26:50:24 | 0 | 0 | 0 |
| | | | |
| C₅/C₉ TM 3:27:70* | 0,5 | 1 | 1 |
| C₅/C₉ TM 13:46:41* | 0 | 0 | 0 |
| C₅/C₉ TM 24:51:25* | 0 | 0 | 0 |
| C₅/C₉ TM 44:45:11* | 0 | 0 | 0 |
| | | | |
| C₅/C₁₀ TM 53:40:7 | 0 | 0 | 0 |
| C₅/C₁₀ TM 36:49:15 | 0 | 0 | 0 |
| C₅/C₁₀ TM 18:50:32 | 0 | 0 | 0 |
| | | | |
| C₅/C₁₃ TM 49:44:7 | 0 | 0 | 0 |
| C₅/C₁₃ TM 29:52:19 | 0 | 0,5 | 0,5 |
| C₅/C₁₃ TM 13:51:36 | 0,5 | 0,5 | 1 |
| | | | |
| C₈/C₉ TM 7:38:55 | 0 | 1 | 1 |
| C₈/C₉ TM 20:46:34 | 0 | 0,5 | 0 |
| C₈/C₉ TM 38:48:14 | 0 | 0.5 | 0 |
| | | | |
| C₈/C₁₀ TM 7:38:55 | 2 | 2,5 | 2,5 |
| C₈/C₁₀ TM 17:49:34 | 1,5 | 2 | 2 |
| C₈/C₁₀ TM 35:48:17 | 1 | 1,5 | 1,5 |
| | | | |
| C₄/C₉ Blend 10:90 | 0 | 0,5 | 0,5 |
| C₄/C₉ Blend 20:80 | 0 | 0,5 | 0 |
| C₄/C₉ Blend 40:60 | 0 | 0 | 0 |
| C₄/C₉ Blend 60:40 | 0 | 0 | 0 |
| C₄/C₉ Blend 80:20 | 0 | 0 | 0 |
| | | | |
| C₅/C₉ Blend 80:20 | 0 | 0 | 0 |
| C₅/C₉ Blend 60:40 | 0 | 0 | 0 |
| C₅/C₉ Blend 40:60 | 0 | 0 | 0 |
| C₅/C₉ Blend 20:80 | 0 | 1 | 0,5 |

| | | | |
|---|---|---|---|
| * erfindungsgemäß | | | |

Die in der Tabelle 13 zusammengestellten Bewertungen zeigen, dass Estergemische, welche C₈- und C₁₀-Alkoholreste oder C₈- und C₉-Alkoholreste oder C₅- und C₁₃-Alkoholreste enthalten, schlecht mit Polymeren, hier PVC, verträglich sind, wenn nennenswerte Mengen des langkettigen Alkoholrestes enthalten sind. Solche Systeme kommen für eine "performanceorientierte" Verarbeitung mit Polymeren deshalb nicht in Betracht

### Beispiel 13:

### Änderung der Masse von Probenkörpern nach Lagerung im Wasser

Die Alterungsbeständigkeit unter verschiedenen Umgebungsbedingungen ist ein weiteres wesentliches Qualitätskriterium für Weichmacher. Insbesondere das Verhalten gegenüber Wasser (Wasseraufnahme & Auswaschverhalten von Rezepturbestandteilen) und gegenüber erhöhten Temperaturen (Ausdunstung von Rezepturbestandteilen und thermische Alterung) bietet einen Einblick in die Alterungsbeständigkeit.

Nimmt ein Kunststoffartikel in größerem Umfang Wasser auf, so verändern sich dadurch einerseits seine Materialeigenschaften, andererseits auch seine Optik (z.B. Eintrübung). Eine hohe Wasseraufnahme ist demnach in der Regel unerwünscht. Das Auswaschverhalten ist ein zusätzliches Kriterium für die Permanenz der Formulierungsbestandteile unter Gebrauchsbedingungen. Dies gilt insbesondere für Stabilisatoren, Weichmacher und/oder ihre Bestandteile, da eine Konzentrationsminderung im Kunststoffartikel bei diesen Rezepturbestandteilen sowohl die Materialeigenschaften verschlechtern als auch die Lebensdauer dramatisch reduzieren kann.

Für die Bestimmung der Wasserbeständigkeit wurden Folien, wie in Beispiel 10 hergestellt, verwendet. Als Prüfkörper wurden Folienkreise mit 3 cm Durchmesser ausgeschnitten. Vor der Wasserlagerung wurden die Prüfkörper für 24 Stunden in einem mit Trockenmittel (KC-Trockenperlen, Fa. BASF SE) ausgerüsteten Exsikkator bei 25 °C gelagert. Das Ausgangsgewicht (Einwaage) wurde mit einer Analysenwaage auf 0,1 mg genau bestimmt. Die Prüfkörper wurden nun in einem mit vollentsalztem (VE) Wasser gefülltem Schüttelbad (Typ "WNB 22" mit Peltierkühlvorrichtung "CDP"; Fa. Memmert GmbH) bei einer Temperatur von 30 °C für 7 Tage mit Probenhaltern unter der Wasseroberfläche gelagert und kontinuierlich bewegt. Nach der Lagerung wurden die Kreise dem Wasserbad entnommen, abgetrocknet und ausgewogen (= Gewicht nach 7 Tagen). Aus der Differenz zur Einwaage wurde die Wasseraufnahme berechnet. Nach der Auswaage wurden die Prüfkörper wiederum für 24 Stunden in einem mit Trockenmittel (KC-Trockenperlen) ausgerüsteten Exsikkator bei 25 °C gelagert und anschließend nochmals ausgewogen (Endauswage = Gewicht nach Trocknung). Aus der Differenz zur Einwaage vor der Wasserlagerung wurde der prozentuale Massenverlust durch Wasserlagerung (entspricht Verlust durch Auswaschungen) berechnet.

**Tabelle 14: Änderung der Masse von Probenkörpern nach Lagerung im Wasser**

| Weichmacher | Massenveränderung nach Trocknung | Weichmacher | Massenveränderung nach Trocknung |
|---|---|---|---|
| DEHT | 0,06 | C₈/C₉ TM 7:38:55 | -0,06 |
| DINT | 0,07 | C₈/C₉ TM 20:46:34 | -0,04 |
| DPT | -0,02 | C₈/C₉ TM 38:48:14 | -0,03 |
| | | | |
| C₄/C₈ TM 3:27:70 | -0,02 | C₈/C₁₀ TM 7:38:55 | 0,08 |
| C₄/C₈ TM 11:45:24 | -0,12 | C₈/C₁₀ TM 17:49:34 | 0,08 |
| C₄/C₈ TM 26:50:24 | -0,23 | C₈/C₁₀ TM 35:48:17 | 0,06 |
| | | | |
| C₅/C₉ TM 3:27:70* | 0,06 | C₄/C₉ Blend 10:90 | -0,10 |
| C₅/C₉ TM 13:46:41* | 0,03 | C₄/C₉ Blend 20:80 | -0,17 |
| C₅/C₉ TM 24:51:25* | 0,05 | C₄/C₉ Blend 40:60 | -0,35 |
| C₅/C₉ TM 44:45:11* | 0,01 | C₄/C₉ Blend 60:40 | -0,49 |
| | | C₄/C₉ Blend 80:20 | -0,65 |
| C₅/C₁₀ TM 53:40:7 | -0,06 | | |
| C₅/C₁₀ TM 36:49:15 | -0,04 | C₅/C₉ Blend 80:20 | -0,04 |
| C₅/C₁₀ TM 18:50:32 | -0,03 | C₅/C₉ Blend 60:40 | -0,02 |
| | | C₅/C₉ Blend 40:60 | 0,00 |
| C₅/C₁₃ TM 49:44:7 | -0,10 | C₅/C₉ Blend 20:80 | 0,00 |
| C₅/C₁₃ TM 29:52:19 | -0,09 | | |
| C₅/C₁₃ TM 13:51:36 | -0,45 | | |

| | | | |
|---|---|---|---|
| * erfindungsgemäß | | | |

Die in der Tabelle 14 zusammengestellten Massenveränderungen zeigen, dass Estergemische, welche C₄- Alkoholreste enthalten, größere Massenverluste bei der Wasserlagerung aufweisen. Die erfindungsgemäßen Gemische hingegen weisen zu vernachlässigende Massenveränderungen nach Lagerung der Prüfkörper in Wasser auf. Eine gute Beständigkeit bei der Lagerung im Wasser ist insbesondere für den Einsatz von Weichmachern in Schläuchen und Außenanwendungen wichtig.

### Beispiel 14:

### Bestimmung der Effizienz der Gemische (Shore A Härte der Weich PVC Proben)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore-Härte. Da in der Praxis Formulierungen / Rezepturen häufig auf eine bestimmte Shore-Härte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet.

Zur Bestimmung der Shore-Härten wurden die im Beispiel 8 hergestellten Plastisole in runde Gießformen aus Messing mit einem Durchmesser von 42 mm gegossen (Einwaage: 20,0 g). Dann wurden die Pasten in den Formen im Umlufttrockenschrank 30 min bei 200 °C geliert, nach Abkühlung entnommen und vor der Messung mindestens 24 Stunden im Klimaschrank (25 °C) gelagert. Die Dicke der Scheiben betrug ca. 12 mm.

Die Härte-Messungen wurden nach DIN 53 505 mit einem Shore-A-Messgerät der Fa. Zwick-Roell durchgeführt, der Messwert jeweils nach 3 Sekunden abgelesen. An jedem Prüfkörper wurden Messungen an drei verschiedenen Stellen durchgeführt, und der Mittelwert gebildet.

**Tabelle 15: Shore A Härte von Prüfkörpern enthaltend die in Tabelle 9 gelisteten Weichmacher**

| Weichmacher | Shore A Härte | Weichmacher | Shore A Härte |
|---|---|---|---|
| DEHT | 82 | C₈/C₉ TM 7:38:55 | 87 |
| DINT | 88 | C₈/C₉ TM 20:46:34 | 86 |
| DPT | 72 | C₈/C₉ TM 38:48:14 | 85 |
| | | | |
| C₄/C₈ TM 3:27:70 | 80 | C₈/C₁₀ TM 7:38:55 | 92 |
| C₄/C₈ TM 11:45:24 | 77 | C₈/C₁₀ TM 17:49:34 | 90 |
| C₄/C₈ TM 26:50:24 | 74 | C₈/C₁₀ TM 35:48:17 | 88 |
| | | | |
| C₅/C₉ TM 3:27:70* | 84 | C₄/C₉ Blend 10:90 | 85 |
| C₅/C₉ TM 13:46:41* | 82 | C₄/C₉ Blend 20:80 | 81 |
| C₅/C₉ TM 24:51:25* | 80 | C₄/C₉ Blend 40:60 | 77 |
| C₅/C₉ TM 44:45:11* | 77 | C₄/C₉ Blend 60:40 | 72 |
| | | C₄/C₉ Blend 80:20 | 68 |
| C₅/C₁₀ TM 53:40:7 | 75 | | |
| C₅/C₁₀ TM 36:49:15 | 78 | C₅/C₉ Blend 80:20 | 75 |
| C₅/C₁₀ TM 18:50:32 | 82 | C₅/C₉ Blend 60:40 | 77 |
| | | C₅/C₉ Blend 40:60 | 79 |
| C₅/C₁₃ TM 49:44:7 | 79 | C₅/C₉ Blend 20:80 | 83 |
| C₅/C₁₃ TM 29:52:19 | 85 | | |
| C₅/C₁₃ TM 13:51:36 | 91 | | |

| | | | |
|---|---|---|---|
| * erfindungsgemäß | | | |

Prüfkörper der erfindungsgemäßen Gemische zeigen eine geringere Shore A Härte und damit eine bessere Weichmacher-Effizienz als Prüfkörper, welche Estergemische umfassend C₈- und C₉-Alkoholreste beziehungsweise C₉- und C₁₀-Alkoholreste, was für die erfindungsgemäßen Gemische zu geringeren Rezepturkosten führt.

## Patentansprüche

1. Gemisch umfassend die Terephthalsäurediester I, II und III, 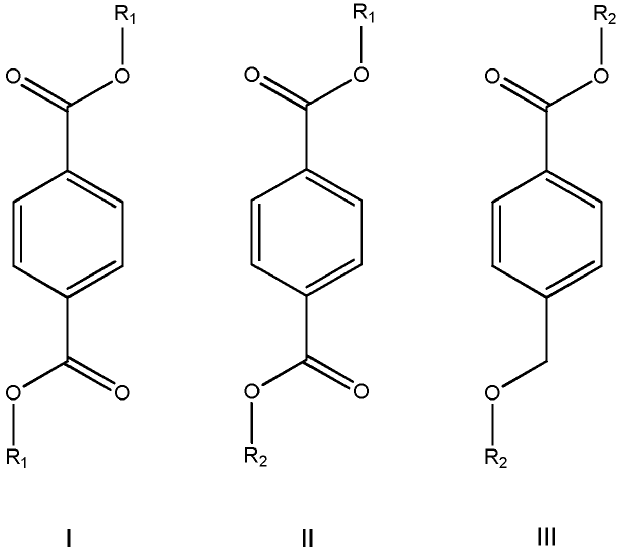 wobei R₁ für einen Alkylrest mit 5 Kohlenstoffatomen und R₂ für einen Alkylrest mit 9 Kohlenstoffatomen steht.

2. Gemisch gemäß Anspruch 1, **dadurch gekennzeichnet, dass** in den Formeln I, II und III R₁ für 2-Methylbutyl-, 3-Methylbutyl-, n-Pentyl- und/oder *Iso*pentyl-Reste und R₂ für Nonyl-, n-Nonyl- und/oder *Iso*nonyl-Reste steht.

3. Gemisch gemäß Anspruch 1 oder 2 , **dadurch gekennzeichnet, dass** das Gemisch weniger als 80 Mol-%, bevorzugt weniger als 70 Mol-% und insbesondere weniger als 60 Mol-% des Esters III, bezogen auf die Gesamtheit der Ester I, II und III enthält.

4. Gemisch gemäß einem der Ansprüche 1 bis 3, **dadurch gekennzeichnet, dass** das Gemisch weniger als 60 Mol-%, vorzugsweise weniger als 50 Mol-% und insbesondere weniger als 40 Mol-% des Esters I, bezogen auf die Gesamtheit der Ester I, II und III enthält.

5. Verwendung des Gemisches gemäß einem der Ansprüche 1 bis 4 als Weichmacher für Polymere, insbesondere für PVC.

6. Verwendung gemäß Anspruch 5, **dadurch gekennzeichnet, dass** das Gemisch als Weichmacher in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, eingesetzt wird.

7. Mittel, enthaltend ein Gemisch gemäß einem der Ansprüche 1 bis 4 und ein oder mehrere Polymere enthält aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

8. Verfahren zur Herstellung eines Estergemisches gemäß einem der Ansprüche 1 bis 4 durch Verestern von Terephthalsäure oder von einem Terephthalsäurederivat mit einem Gemisch umfassend R₁OH (R₁= Alkylrest mit 5 Kohlenstoffatomen) und R₂OH (R₂= Alkylrest mit 9 Kohlenstoffatomen).

9. Verfahren zur Herstellung eines gemäß einem der Ansprüche 1 bis 4, in welchem der Ester I mit weniger als 1 Mol-Äquivalent, bezogen auf die Anzahl seiner Esterfunktionen, eines Alkohols oder mehrerer Alkohole mit 9 Kohlenstoffatomen umgesetzt und dabei vorzugsweise zum Sieden erhitzt wird.

10. Verfahren gemäß Anspruch 9, **dadurch gekennzeichnet, dass** der Ester I mit einem Alkohol oder mehreren Alkoholen mit 9 Kohlenstoffatomen in Anwesenheit eines Katalysators umgesetzt und dabei zum Sieden erhitzt wird, wobei mehr als 80 Mol-%, vorzugsweise mehr als 90 Mol-% und insbesondere mehr als 95 Mol-% des sich im Laufe des Verfahrens bildenden C₅-Alkohols während des Verfahrens - vorzugsweise mittels Destillation - aus dem Reaktionsgefäß entfernt werden.

11. Verfahren zur Herstellung eines gemäß einem der Ansprüche 1 bis 4 durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als der Alkohol R₁OH des Restes R₁ bei demselben Druck, mit einer Menge (m₁+ s₁) R₁OH und einer Menge m₂ R₂OH, mit einer Menge (m₁+ s₁) R₁OH und einer Menge m₂ R₂OH, wobei das Reaktionsgemisch zum Sieden erhitzt wird und
m₁ und m₂ den Mol-Äquivalenten der in Terephthalsäure oder deren Derivaten einzuführenden Alkoholresten OR₁ (C₅-Alkoholrest) und OR₂ (C₉-Alkoholrest) entspricht und
s₁ größer als 0 ist.

12. Verfahren gemäß Anspruch 11, **dadurch gekennzeichnet, dass** die Menge s₁ kleiner als (m₁ + m₂), mit Vorzug kleiner als 0,6 · (m₁ + m₂), besonders bevorzugt kleiner als 0,5 · (m₁ + m₂) und insbesondere kleiner als 0,4 · (m₁ + m₂) und gleichzeitig bevorzugt größer als 0,05 · (m₁ + m₂), besonders bevorzugt größer als 0,10· (m₁ + m₂) und insbesondere größer als 0,15 · (m₁ + m₂) ist.

13. Verfahren gemäß einem der Ansprüche 11 oder 12, **dadurch gekennzeichnet, dass** mindestens 0,8 Mol-Äquivalente, bevorzugt mindestens 0,9 Mol-Äquivalente und insbesondere mindestens 0,95 Mol-Äquivalente eingesetzten Alkohols R₂OH als OR₂-Rest in die Terephthalsäure oder deren Derivat eingeführt wird und/oder dass in dem Verfahren ein Katalysator eingesetzt wird und im Reaktionsgemisch der Gehalt an C₅-Alkohol auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des C₅-Alkohol - abgesenkt wurde, bevor der Katalysator zerstört wird.

14. Verfahren gemäß einem der Ansprüche 8 bis 13, **dadurch gekennzeichnet, dass** R₁ für 2-Methylbutyl-, 3-Methylbutyl-, *n*-Pentyl- und/oder *Iso*pentyl-Reste und R₂ für einen *Iso*nonyl-Rest steht, wobei das Gemisch vorzugsweise weniger als 80 Mol-%, bevorzugt weniger als 70 Mol-% und insbesondere weniger als 60 Mol-% des Esters III, bezogen auf die Gesamtheit der Ester I, II und III enthält.

15. Verwendung der Herstellungsverfahren II und III zur Einstellung verarbeitungs- und/oder anwendungsrelevanter Eigenschaften eines Estergemisches durch Steuerung der Mengenverteilung der Ester im Estergemisch.
